# EUROPEAN PATENT APPLICATION

(11) **EP 3 431 495 A1**
(43) Date of publication of application: **23.01.2019**
(21) Application number: 18177236.9
(22) Date of filing: 19.10.2012
(51) Int. Cl.: C07K 16/00, C07K 16/46, A61K 39/395, C07K 16/22, C07K 16/24

(54) **STABLE MULTIPLE ANTIGEN-BINDING ANTIBODY**

(30) Priority: 20.10.2011 US 201161549482 P
(62) Divisional of application: 12780659.4
(71) Applicant: ESBATech - a Novartis Company LLC, 8952 Schlieren (CH)
(72) Inventor: Riegler, Astrid C., 90489 Nürnberg (DE); Borras, Leonardo, 8952 Schlieren (CH); Sommavilla, Roberto, 8152 Opfikon (CH)
(74) Representative: Grünecker Patent- und Rechtsanwälte PartG mbB

(57) **Abstract**

The invention provides antibodies that bind to multiple antigens, said antibodies having at least two antibody light chain variable domains and two antibody heavy chain variable domains, wherein each light chain variable domain is linked to a heavy chain variable domain to form a VH/VL construct, and wherein at least one of the VH domains comprises a particular amino acid at AHo position 12, 103 and/or 144, and at least one of the VL domains comprises a particular amino acid at AHo position 47 and/or 50. Nucleic acid molecules, vectors and host cells for expression of the recombinant antibodies of the invention, methods for isolating them, and the use of said antibodies in medicine are also disclosed.

## Description

### CROSS-REFERENCE TO RELATED APPLICATION

This application claims priority under 35 U.S.C. §119 to U.S. Provisional Patent Application No. 61/549,482 filed October 20, 2011, the entire contents of which are incorporated herein by reference.

### Field of the Invention

The invention relates to a stable multiple antigen-binding antibody having at least two antibody light chain variable domains, two antibody heavy chain variable domains, and lacks constant domains, wherein each light chain variable domain is linked to a heavy chain variable domain to form a VH/VL construct, and wherein at least one of the VH domains comprises a particular amino acid at AHo position 12, 103 and/or 144, and at least one of the VL domains comprises a particular amino acid at AHo position 47 and/or 50. The invention further relates to methods of producing such antibodies, and pharmaceutical compositions comprising such antibodies.

### Background of the Invention

Antibody molecules that are capable of binding to more than one antigen are desirable as potential therapeutic agents for treating diseases involving multiple proteins. For example, it is often desirable to target two proteins in the same signaling pathway or to modulate activity of two different pathways by targeting a protein in each pathway. Examples of such antibodies include multi-specific antibodies (e.g., bispecific antibodies that bind two different target molecules) and multivalent antibodies (e.g., bivalent antibodies that bind two different binding sites of one target molecule). Several approaches have been developed in the field of therapeutic antibodies to combine two therapeutic antibodies into a single molecule to take advantage of additive or synergistic effects while maintaining stability and other desirable properties. Such approaches include recombinant formats such as the tandem single-chain variable fragment (TdscFv) (Hagemeyer et al., 2009, Thromb Haemost 101:1012-1019; Robinson et al, 2008, Br J Cancer 99:1415-1425), diabodies (Hudson et al., 1999, J Immunol Methods 231:177-189), tandem diabodies (Kipriyanov, 2009, Methods Mol Biol 562:177-193), two-in-one antibodies (Bostrom et al., 2009, Science 323:1610-1614), and dual variable domain antibodies (Wu et al., 2007, Nat Biotechnol 25:1290-1297).

One example of a disease in which a two-component therapeutic approach is attractive is choroidal neovascularization. The vascular component of choroidal neovascularization is comprised of vascular endothelial cells, endothelial cell precursors, and pericytes. The extravascular component, which by histopathology appears to be both the source of angiogenic stimuli and often the largest component volumetrically, is comprised of inflammatory, glial and retinal pigment epithelial cells, and fibroblasts. Tissue damage can be caused by either component. Each component can be targeted separately through a variety of monotherapies. However, a bispecific antibody against a vascular endothelial growth factor (VEGF) and a tumor necrosis factor (TNF) represent an opportunity of attacking both components simultaneously.

A common problem associated with multi-specific and multivalent antibodies is poor stability, as well as problems with production yield, purity, and affinity. Various approaches to address these problems have been developed, including rational design and directed evolution (Mabry and Snavely, 2010, IDrugs 13:543-549). Such approaches, however, take time and have yet to provide universally applicable results.

Consequently, there is a need in the art for a multi-specific and multivalent antibody format that is stable and soluble, and lacks the deficiencies of traditional multi-specific and multivalent antibody formats.

### Summary of the Invention

The invention provides antibodies that bind to multiple antigens, such as bispecific and bivalent antibodies, comprising certain amino acid residues at particular positions in a variable heavy and/or variable light chain, that are highly stable molecules.

In one aspect, the invention provides multiple antigen-binding antibody molecules comprising:
a) two heavy chain variable domains, one with specificity to antigen A (VH-A) and one with specificity for antigen B (VH-B), and
b) two light chain variable domains, one with specificity to antigen A (VL-A) and one with specificity for antigen B (VL-B),
wherein at least one of the two heavy chain variable domains comprises at least one of the following: a Serine at AHo position 12, a Serine or Threonine at AHo position 103, and a Serine or Threonine at AHo position 144; and/or wherein at least one of the two light chain variable domains comprises an Arginine at AHo position 50.

In certain aspects, the VH-A is linked to VL-A to form a single chain antibody with specificity for antigen A (scFv A) and VH-B is linked to VL-B to form a single chain antibody with specificity for antigen B (scFv B).

In one aspect, the invention provides a multiple antigen-binding antibody molecule comprising: a heavy chain variable domain with a specificity for antigen A (VH-A) linked to a light chain variable domain with a specificity for antigen B (VL-B) by peptide linker 1 to form a VH-A/VL-B construct; a heavy chain variable domain with a specificity for antigen B (VH-B) linked to a light chain variable domain with a specificity for antigen A (VL-A) by peptide linker 2 to form a VH-B/VL-A construct; wherein the multiple antigen-binding antibody lacks constant domains, at least one of VL-A and VL-B comprises an Arginine at AHo position 50, and at least one of VH-A and VH-B comprises at least one of the following: a Serine at AHo position 12, a Serine or Threonine at AHo position 103, and a Serine or Threonine at AHo position 144.

In certain aspects, a VH-A/VL-B construct is in a VH-A-(linker 1)-VL-B orientation or VH-B-(linker 1)-VL-A orientation. In other aspects, a VH-B/VL-A construct is in a VH-B-(linker 2)-VL-A orientation or VH-A-(linker 2)-VL-B orientation.

In another aspect, at least one of VL-A or VL-B comprises a framework sequence that has at least 65% identity to the sequence of SEQ ID NO: 6.

In another aspect, at least one of VH-A or VH-B comprises a framework sequence that has at least 80% identity to the sequence of SEQ ID NO: 7.

In another aspect, at least one of the VL-A/VH-B and the VH-B/VL-A constructs comprises a human Vkappa1 family light chain variable region, a human Vlambda 1 family light chain variable region, or a human Vkappa3 family light chain variable region.

In another aspect, at least one of the VL-A/VH-B and the VH-B/VL-A constructs comprises a human VH3 family heavy chain variable region, a human VHla family heavy chain variable region, or a human VH1b family heavy chain variable region.

In another aspect, the VH domains and the VL domains comprise CDRs from a lagomorph.

In another aspect, the invention further provides a multiple antigen-binding antibody comprising:
a) a single-chain antibody comprising a heavy chain variable domain with a specificity for antigen A (VH-A) linked to a light chain variable domain with a specificity for antigen A (VL-A) by peptide linker 3 to form scFv-A;
b) a single-chain antibody comprising a heavy chain variable domain with a specificity for antigen B (VH-B) linked to a light chain variable domain with a specificity for antigen B (VL-B) by peptide linker 3 to form scFv-B;
wherein scFv-A is linked to scFv-B by a peptide linker 1, and at least one of VL-A and VL-B comprises an Arginine at AHo position 50, and at least one of VH-A and VH-B comprises at least one of the following: Serine at AHo position 12, a Serine or Threonine at AHo position 103, and a Serine or Threonine at AHo position 144.

In one aspect, a multiple antigen-binding antibody of the invention comprises a single-chain antibody with specificity for antigen A and a single-chain antibody with specificity for antigen B in the following format: VH-A/VL-A - linker - VH-B/VL-B. In a preferred aspect the linker has 20 amino acids. In another preferred aspect, the linker has the sequence of SEQ ID NO: 4.

In yet another aspect, the invention provides a multiple antigen-binding antibody molecule comprising: CDRs from a lagomorph; a heavy chain variable domain with a specificity for antigen A (VH-A) linked to a light chain variable domain with a specificity for antigen B (VL-B) by peptide linker 1 to form a VH-A/VL-B construct; a heavy chain variable domain with a specificity for antigen B (VH-B) linked to a light chain variable domain with a specificity for antigen A (VL-A) by peptide linker 2 to form a VH-B/VL-A construct; wherein at least one of the heavy chain variable domains comprises at least three of the following: threonine (T) at AHo position 24, valine (V) at AHo position 25, alanine (A) or glycine (G) at AHo position 56, lysine (K) at AHo position 82, threonine (T) at AHo position 84, valine (V) at AHo position 89 and arginine (R) at AHo position 108. In certain aspects, such antibodies further comprise glutamic acid (E) at AHo position 1, valine (V) at AHo position 3, leucine (L) at AHo position 4, Serine (S) at AHo position 10; Arginine (R) at AHo position 47, Serine (S) at AHo position 57, phenylalanine (F) at AHo position 91 and/or Valine (V) at AHo position 103 in at least one of the variable light chain domains:

Specific preferred embodiments of the invention will become evident from the following more detailed description of certain preferred embodiments and the claims.

### Detailed Description

The particulars shown herein are by way of example and for purposes of illustrative discussion of the preferred embodiments of the present invention only and are presented in the cause of providing what is believed to be the most useful and readily understood description of the principles and conceptual aspects of various embodiments of the invention. In this regard, no attempt is made to show structural details of the invention in more detail than is necessary for the fundamental understanding of the invention, the description taken with the drawings and/or examples making apparent to those skilled in the art how the several forms of the invention may be embodied in practice.

In order that the present invention may be more readily understood, certain terms are defined as follows and as set forth throughout the detailed description. The definitions and explanations are meant and intended to be controlling in any future construction unless clearly and unambiguously modified in the following examples or when application of the meaning renders any construction meaningless or essentially meaningless. In cases where the construction of the term would render it meaningless or essentially meaningless, the definition should be taken from Webster's Dictionary, 3rd Edition or a dictionary known to those of skill in the art, such as the Oxford Dictionary of Biochemistry and Molecular Biology (Ed. Anthony Smith, Oxford University Press, Oxford, 2004).

It is a general object of the invention to provide antibodies capable of binding multiple antigens that are suitable for therapeutic uses. As described herein for the first time, properties of such antibodies can be improved when certain amino acids are present at particular positions in the light chain variable domain and heavy chain variable domain. Such improved properties include increased stability, production yield, and purity, for example.

Therefore, the invention provides antibodies that bind multiple antigens, the antibodies comprising:
a) a heavy chain variable domain with a specificity for antigen A (VH-A) linked to a light chain variable domain with a specificity for antigen B (VL-B) by peptide linker 1 to form a VH-A/VL-B construct;
b) a heavy chain variable domain with a specificity for antigen B (VH-B) linked to a light chain variable domain with a specificity for antigen A (VL-A) by peptide linker 2 to form a VH-B/VL-A construct;
wherein at least one of VL-A and VL-B comprises an Arginine at AHo position 47 and/or 50, and at least one of VH-A and VH-B comprises a Serine at AHo position 12, a Serine or Threonine at AHo position 103, and/or a Serine or Threonine at AHo position 144. In one embodiment, a VL comprises Arginine at AHo position 50, and a VH comprises Serine at AHo position 12, Threonine at AHo position 103, and Threonine at AHo position 144.

In a preferred embodiment, an antibody of the invention is a multiple antigen-binding antibody that lacks constant domains, such as a scFv antibody.

The term "scFv" refers to a molecule comprising an antibody heavy chain variable domain (or region; VH) and an antibody light chain variable domain (or region; VL) connected by a linker, and lacks constant domains. Such scFv molecules can have the general structures: NH2-VL-linker-VH-COOH or NH2-VH-linker-VL-COOH. Suitable linkers are described herein and are known to those of skill in the art, including linkers described, for example, in International Patent Application WO 2010/006454.

As used herein, a "multiple antigen-binding antibody" is an antibody that has at least four variable domains and can bind two or more antigens of different target molecules (e.g., a bispecific antibody) or two or more antigens of the same target molecule (e.g., a bivalent antibody). Forms of multiple antigen-binding antibodies of the invention include, but are not limited to, a diabody, a single-chain diabody, and a tandem antibody, as known to those of skill in the art.

A "bispecific antibody" as used herein is an antibody that can bind two different target molecules.

A "bivalent antibody" as used herein is an antibody that can bind two different sites of one target molecule.

In certain embodiments, a VL of a multiple antigen-binding antibody of the invention comprises at least one particular amino acid in at least one particular position that has been shown herein to improve stability of a multiple antigen-binding antibody format, and the VH of the multiple antigen-binding antibody of the invention comprises at least one of three particular amino acids in at least one of three particular positions that have been shown herein to improve stability of a multiple antigen-binding antibody format. In a particular embodiment, the position in the VL is AHo position 50 and/or AHo position 47, and the amino acid at said position(s) is an Arginine. In another particular embodiment, the positions in the VH are AHo positions 12, 103, and 144, and the preferred amino acids at said positions are: Serine at AHo position 12, a Serine or Threonine at AHo position 103, and a Serine or Threonine at AHo position 144. In a preferred embodiment, the amino acids in the VH are Serine at AHo position 12, Threonine at AHo position 103, and Threonine at AHo position 144. In certain embodiments, these preferred amino acids can be introduced into a VL and/or VH by substitution of the naturally occurring amino acid at the identified position(s).

The AHo numbering system is described further in Honegger, A. and Pluckthun, A. (2001) J. Mol. Biol. 309:657-670). Alternatively, the Kabat numbering system as described further in Kabat *et al.* (Kabat, E. A., et al. (1991) Sequences of Proteins of Immunological Interest, Fifth Edition, U.S. Department of Health and Human Services, NIH Publication No. 91-3242) may be used. Conversion tables for the two different numbering systems used to identify amino acid residue positions in antibody heavy and light chain variable regions are provided in A. Honegger, 2001, J.Mol.Biol. 309:657-670. The corresponding Kabat number for AHo position 47 in the VL is 39. The corresponding Kabat number for AHo position 50 in the VL is 42. The corresponding Kabat number for AHo position 12 in the VH is 11. The corresponding Kabat number for AHo position 103 in the VH is 89. The corresponding Kabat number for AHo position 144 in the VH is 108.

In another embodiment, the invention provides multiple antigen-binding antibodies comprising one or more of the preferred amino acids at the preferred positions disclosed herein, and that comprise binding specificities of at least two antibodies, or antibody fragments thereof, including, *e.g*., an Fab, Fab', F(ab')₂, Fv, or a single chain Fv. Such antibodies may also be a light chain or heavy chain dimer, or any minimal fragment thereof such as a Fv or a single chain construct as described in Ladner et al. U.S. Patent No. 4,946,778, the contents of which are expressly incorporated by reference. In certain embodiments, such multiple antigen-binding antibodies comprise at least one VL having an Arginine at AHo position 50, and at least one VH having a Serine at AHo position 12, a Serine or Threonine at AHo position 103, and a Serine or Threonine at AHo position 144.

### Linkers and VH/VL Constructs

As used herein, "peptide linker 1" and "peptide linker 2" refer to linker peptides that connect variable domains in a VH/VL construct to each other, or one or more scFv constructs together. A "VH/VL construct" can be: a VH-A/VL-B or VH-B/VL-A construct, which comprises a VH domain with CDRs that bind to a particular antigen and a VL domain with CDRs that bind to a different antigen; or a VH-A/VL-A or VH-B/VL-B construct, which comprise a VH domain with CDRs that bind to a particular antigen and a VL domain with CDRs that bind to the same antigen. The form of such constructs can be VH-L-VL or VL-L-VH, where L is peptide linker 1 or 2. Such peptide linkers are preferably less than or equal to about 20 amino acids long. In particular, such peptide linkers are 1, 2, 3, 4, 5, 6, 7, 8, 9, or 10 amino acids long. In certain embodiments, the peptide linkers are 3-7 amino acids long. A preferred linker sequence for peptide linker 1 and/or peptide linker 2 is GGGGS (SEQ ID NO: 1). Other linker sequences useful as a peptide linker 1 and/or peptide linker 2 in a multiple antigen-binding antibody of the invention include: GGS and GGGGGGS (SEQ ID NO: 2). In certain embodiments a VH/VL construct may be a single-chain antibody. Linkers in single-chain antibodies are known in the art. Preferably, a linker for a single-chain antibody that links a VH and VL domain that bind to a preferred antigen in an antibody of the invention can be up to 20 amino acids, for example having the sequence of SEQ ID NO: 4 shown below.

In certain embodiments, a VH/VL construct is connected to another VH/VL construct by peptide linker 3. In other embodiments, variable domains in a VH/VL construct are connected to each other by a peptide linker 3. Peptide linker 3 is preferably more than about 10 amino acids and less than about 30 amino acids long. In particular, peptide linker 3 is 10, 11, 12, 13, 14, 15, 16, 17, 18, 19, or 20 amino acids long. In certain embodiments, peptide linker 3 is 10-15 amino acids long. A preferred linker sequence for peptide linker 3 is GSDSNAGRASAGNTS (SEQ ID NO: 3). Another preferred linker sequence for peptide linker 3 is (GGGGS)₄ (SEQ ID NO: 4). One of skill in the art will recognize that conservative changes (e.g., substitutions) can be made to linker sequences without affecting the activity and preferred properties of a multiple antigen-binding antibody of the invention.

In certain embodiments, a VH/VL construct can be in one of the following formats: VH-A-(linker 1 or 2)-VL-B; VH-B-(linker 1 or 2)-VL-A; VH-A-(linker 1 or 2)-VL-A; VH-B-(linker 1 or 2)-VL-B; VL-A-(linker 1 or 2)-VH-A; VL-B-(linker 1 or 2)-VH-B. Other orientations of the VH and VL domains in a VH/VL construct can be contemplated by those of skill in the art. For example, VL-A-(linker 1 or 2)-VH-B, VL-B-(linker 1 or 2)-VH-A, VL-A-(linker 1 or 2)-VH-A, VL-B-(linker 1 or 2)-VH-B. In particular embodiments, where VH/VL constructs are connected by peptide linker 3, one of the following formats may be created: VH-A-(linker-l)-VL-A-(linker 3)-VH-B-(linker 2)-VL-B; VH-A-(linker-1)-VL-B-(linker 3)-VH-B-(linker 2)-VL-A; VL-A-(linker-l)-VH-B-(linker 3)-VL-B-(linker 2)-VH-A, VL-A-(linker-1)-VH-A-(linker 3)-VL-B-(linker 2)-VH-B. The formats identified herein are non-limiting examples, and it should be readily understood that those of skill in the art may arrange the VH and VL domains in various other orientations, so long as binding of the target antigens is accomplished when the constructs are produced and properly folded.

In one embodiment, a multiple antigen-binding antibody of the invention comprises a single-chain antibody with specificity for antigen A and a single-chain antibody with specificity for antigen B in the following format: VH-A/VL-A - linker 3 - VH-B/VL-B, wherein the linker 3 has the sequence of SEQ ID NO: 4.

In one embodiment, a preferred format of a multiple antigen-binding antibody of the invention is: VH-A-SEQ ID NO: 1-VL-B-SEQ ID NO: 4-VH-B-SEQ ID NO: 1-VL-A.

Alternatively, VH/VL constructs of the invention can be functionally linked (*e.g*., by chemical coupling, genetic fusion, noncovalent association or otherwise) to one another to form a multiple antigen-binding antibody.

The invention provides antibodies that bind multiple antigens. As described herein, such antibodies may bind to different target molecules (e.g., a bispecific antibody having specificity for at least two different proteins) or bind to different epitopes on the same target molecule (e.g., a bivalent antibody having specificity for the same protein but binding two or more binding sites on that protein). The particular target molecule(s) can be selected by one of skill in the art depending on the need.

By way of example, and not limitation, the invention provides a bispecific antibody that has specificity for VEGF and TNFα as described in the Examples herein. Such an antibody is useful for treating diseases in which it is desirable to inhibit VEGF and TNFα.

In certain embodiments, an anti-VEGF/TNFa antibody may be used in the treatment of age-related macular degeneration, choroidal neovascularization, neovascular glaucoma, diabetic retinopathy, retinopathy of prematurity, retrolental fibroplasia, breast carcinomas, lung carcinomas, gastric carcinomas, esophageal carcinomas, colorectal carcinomas, liver carcinomas, ovarian carcinomas, the comas, arrhenoblastomas, cervical carcinomas, endometrial carcinoma, endometrial hyperplasia, endometriosis, fibrosarcomas, choriocarcinoma, head and neck cancer, nasopharyngeal carcinoma, laryngeal carcinomas, hepatoblastoma, Kaposi's sarcoma, melanoma, skin carcinomas, hemangioma, cavernous hemangioma, hemangioblastoma, pancreas carcinomas, retinoblastoma, astrocytoma, glioblastoma, Schwannoma, oligodendroglioma, medulloblastoma, neuroblastomas, rhabdomyosarcoma, osteogenic sarcoma, leiomyosarcomas, urinary tract carcinomas, thyroid carcinomas, Wilm's tumor, renal cell carcinoma, prostate carcinoma, abnormal vascular proliferation associated with phakomatoses, edema (such as that associated with brain tumors), Meigs' syndrome, rheumatoid arthritis, psoriasis, atherosclerosis, chronic and/or autoimmune states of inflammation in general, immune mediated inflammatory disorders in general, inflammatory CNS disease, inflammatory diseases affecting the eye, joint, skin, mucuous membranes, central nervous system, gastrointestinal tract, urinary tract or lung, states of uveitis in general, retinitis, HLA-B27+ uveitis, Behcet's disease, dry eye syndrome, glaucoma, Sjögren syndrome, diabetes mellitus (incl. diabetic neuropathy), insulin resistance, states of arthritis in general, rheumatoid arthritis, osteoarthritis, reactive arthritis and Reiter's syndrome, juvenile arthritis, ankylosing spondylitis, multiple sclerosis, Guillain-Barre syndrome, myasthenia gravis, amyotrophic lateral sclerosis, sarcoidosis, glomerulonephritis, chronic kidney disease, cystitis, psoriasis (including psoriatic arthritis), hidradenitis suppurativa, panniculitis, pyoderma gangrenosum, SAPHO syndrome (synovitis, acne, pustulosis, hyperostosis and osteitis), acne, Sweet's sydrome, pemphigus, Crohn's disease (incl. extraintestinal manifestastations), ulcerative colitis, asthma bronchiale, hypersensitivity pneumonitis, general allergies, allergic rhinitis, allergic sinusitis, chronic obstructive pulmonary disease (COPD), lung fibrosis, Wegener's granulomatosis, Kawasaki syndrome, Giant cell arteritis, Churg-Strauss vasculitis, polyarteritis nodosa, burns, graft versus host disease, host versus graft reactions, rejection episodes following organ or bone marrow transplantation, sytemic and local states of vasculitis in general, systemic and discoid lupus erythematodes, polymyositis and dermatomyositis, sclerodermia, pre-eclampsia, acute and chronic pancreatitis, viral hepatitis, alcoholic hepatitis, postsurgical inflammation such as after eye surgery (e.g. cataract (eye lens replacement) or glaucoma surgery), joint surgery (incl. arthroscopic surgery), surgery at joint-related structures (e.g. ligaments), oral and/or dental surgery, minimally invasive cardiovascular procedures (e.g. PTCA, atherectomy, stent placement), laparoscopic and/or endoscopic intra-abdominal and gynecological procedures, endoscopic urological procedures (e.g. prostate surgery, ureteroscopy, cystoscopy, interstitial cystitis), or perioperative inflammation (prevention) in general, Alzheimer disease, Parkinson's disease, Huntington's disease, Bell' palsy, Creutzfeld-Jakob disease. Cancer-related osteolysis, cancer-related inflammation, cancer-related pain, cancer-related cachexia, bone metastases, acute and chronic forms of pain, irrespective whether these are caused by central or peripheral effects of TNFα and whether they are classified as inflammatory, nociceptive or neuropathic forms of pain, sciatica, low back pain, carpal tunnel syndrome, complex regional pain syndrome (CRPS), gout, postherpetic neuralgia, fibromyalgia, local pain states, chronic pain syndroms due to metastatic tumor, dismenorrhea. Bacterial, viral or fungal sepsis, tuberculosis, AIDS, atherosclerosis, coronary artery disease, hypertension, dyslipidemia, heart insufficiency and chronic heart failure.

Binding of antibodies of the invention to their specific target antigens can be confirmed by, for example, enzyme-linked immunosorbent assay (ELISA), radioimmunoassay (RIA), FACS analysis, bioassay (*e.g*., growth inhibition), or by immunoblot assay. Each of these assays generally detects the presence of protein-antibody complexes of particular interest by employing a labeled reagent (*e.g*., an antibody) specific for the complex of interest. Alternatively, the complexes can be detected using any of a variety of other immunoassays. For example, the antibody can be radioactively labeled and used in a radioimmunoassay (RIA) (see, for example, Weintraub, B., Principles of Radioimmunoassays, Seventh Training Course on Radioligand Assay Techniques, The Endocrine Society, March, 1986, which is incorporated by reference herein). The radioactive isotope can be detected by such means as the use of a γ counter or a scintillation counter or by autoradiography.

Antibodies of the invention are useful for a number of purposes, including therapeutic and diagnostic purposes.

### Characteristics of the VL and VH Domains

In certain embodiments, a VL and VH of a multiple antigen-binding antibody of the invention comprises CDRs from a human antibody, a non-human antibody (such as an antibody produced in a rodent, non-human primate, lagomorph, or any other suitable animal), a chimeric antibody, a humanized antibody, and the like. In a particular embodiment, the CDRs are from a lagomorph.

The term "lagomorph" refers to members of the taxonomic order Lagomorpha, comprising the families Leporidae (e.g. hares and rabbits), and the Ochotonidae (pikas). In a most preferred embodiment, the lagomorph is a rabbit. The term "rabbit" as used herein refers to an animal belonging to the family of the leporidae.

The term "CDR" refers to one of the six hypervariable regions within the variable domains of an antibody that mainly contribute to antigen binding. One of the most commonly used definitions for the six CDRs was provided by Kabat E.A. et al. (1991, Sequences of proteins of immunological interest. NIH Publication 91-3242). In some cases, Kabat's definition of CDRs can be applied only for CDR1, CDR2 and CDR3 of the light chain variable domain (CDR L1, CDR L2, CDR L3, or L1, L2, L3), as well as for CDR2 and CDR3 of the heavy chain variable domain (CDR H2, CDR H3, or H2, H3), while CDR1 of the heavy chain variable domain (CDR HI or H1) is defined by the following residues (Kabat numbering): CDR1 of the heavy chain starts with position 26 and ends prior to position 36. This definition is basically a fusion of CDR H1 as differently defined by Kabat and Chothia.

In one embodiment, a VL in a multiple antigen-binding scFv antibody of the invention comprises a sequence having at least 65% identity, more preferably at least 80%, 85%, 90%, 95%, 96%, 97%, 98%, more preferably 99% identity, to the following sequence (SEQ ID NO: 5):

EIVMTQSPSTLSASVGDRVIITC(X)n=1-50 WYQQKPGRAPKLLIY(X)n=1-50
GVPSRFSGSGSGAEFTLTISSLQPDDFATYYC(X)n=1-50 FGQGTKLTVLG

In another embodiment, the VH a multiple antigen-binding scFv antibody of the invention comprises a sequence having at least 80% identity, more preferably at least 85%, 90%, 91%, 92%, 93%, 94%, 95%, 96%, 97%, 98%, more preferably 99% identity, to the following sequence (SEQ ID NO: 6):

EVQLVESGGGLVQPGGSLRLSCTAS(X)n=1-50 WVRQAPGKGLEWVG(X)n=1-50
RFTISRDTSKNTVYLQMNSLRAEDTAVYYCAR(X)n=1-50 WGQGTLVTVSS

As used herein, X residues are CDR insertion sites. X may be any naturally occurring amino acid; at least three and up to 50 amino acids can be present. The framework sequences of SEQ ID NO: 5 and SEQ ID NO: 6 is understood to be the sequences without the X residues.

Suitable antibody frameworks useful in a multiple antigen-binding antibody of the invention include, but are not limited to: a human Vkappa1 family light chain variable region, a human Vlambda 1 family light chain variable region, a human Vkappa3 family light chain variable region, a human VH3 family heavy chain variable region, a human VH1a family heavy chain variable region, and a human VH1b family heavy chain variable region, wherein the light chain variable region has or has been engineered to have (for example by substitution of the naturally occurring amino acid) an Arginine at AHo position 47 and/or 50, and the heavy chain variable region has or has been engineered to have (for example by substitution of the naturally occurring amino acid) a Serine at AHo position 12, a Serine or Threonine at AHo position 103, and a Serine or Threonine at AHo position 144.

Non-limiting examples of frameworks that can be used to generate a multiple antigen-binding antibody of the invention include those frameworks disclosed in International Patent Application WO 2008/004834, International Patent Application WO 2009/155726, and International Patent Application WO 03/097697, the entire contents of which are incorporated by reference. In such examples, the light chain variable region may be modified to include an Arginine at AHo position 47 and/or 50, and the heavy chain variable region may be modified to include a Serine at AHo position 12, a Serine or Threonine at AHo position 103, and/or a Serine or Threonine at AHo position 144.

The term "antibody framework," or "framework," as used herein refers to the part of the variable domain, either VL or VH, which serves as a scaffold for the antigen binding loops (CDRs) of this variable domain. In essence it is the variable domain without the CDRs.

As used herein, "identity" refers to the sequence matching between two polypeptides, molecules or between two nucleic acids. When a position in both of the two compared sequences is occupied by the same base or amino acid monomer subunit (for instance, if a position in each of the two DNA molecules is occupied by adenine, or a position in each of two polypeptides is occupied by a lysine), then the respective molecules are identical at that position. The "percentage identity" between two sequences is a function of the number of matching positions shared by the two sequences divided by the number of positions compared x 100. For instance, if 6 of 10 of the positions in two sequences are matched, then the two sequences have 60% identity. By way of example, the DNA sequences CTGACT and CAGGTT share 50% identity (3 of the 6 total positions are matched). Generally, a comparison is made when two sequences are aligned to give maximum identity. Such alignment can be provided using, for instance, the method of Needleman et al. (1970) J. Mol. Biol. 48: 443-453, implemented conveniently by computer programs such as the Align program (DNAstar, Inc.). The percent identity between two amino acid sequences can also be determined using the algorithm of E. Meyers and W. Miller (Comput. Appl. Biosci., 4:11-17 (1988)) which has been incorporated into the ALIGN program (version 2.0), using a PAM120 weight residue table, a gap length penalty of 12 and a gap penalty of 4. In addition, the percent identity between two amino acid sequences can be determined using the Needleman and Wunsch (J. Mol. Biol. 48:444-453 (1970)) algorithm which has been incorporated into the GAP program in the GCG software package (available at www.gcg.com), using either a Blossum 62 matrix or a PAM250 matrix, and a gap weight of 16, 14, 12, 10, 8, 6, or 4 and a length weight of 1, 2, 3, 4, 5, or 6.

In one embodiment, a multiple antigen-binding antibody of the invention comprises lagomorph CDRs and one or more heavy chain variable domains that comprise three, four, five, six, or seven of the following: threonine (T) at AHo position 24, valine (V) at AHo position 25, alanine (A) or glycine (G) at AHo position 56, lysine (K) at AHo position 82, threonine (T) at AHo position 84, valine (V) at AHo position 89 and arginine (R) at AHo position 108.

In another embodiment, a multiple antigen-binding antibody of the invention comprises lagomorph CDRs and glutamic acid (E) at AHo position 1, valine (V) at AHo position 3, leucine (L) at AHo position 4, Serine (S) at AHo position 10; Arginine (R) at AHo position 47, Serine (S) at AHo position 57, phenylalanine (F) at AHo position 91 and/or Valine (V) at AHo position 103 in at least one of the variable light chain domains.

### Nucleic Acid Molecules and Vectors

In one embodiment, the invention includes nucleic acid molecules for the production of a multiple antigen-binding scFv antibody of the invention.

The term "nucleic acid molecule," refers to DNA molecules and RNA molecules. A nucleic acid molecule may be single-stranded or double-stranded, but preferably is double-stranded DNA. A nucleic acid is "operably linked" when it is placed into a functional relationship with another nucleic acid sequence. For instance, a promoter or enhancer is operably linked to a coding sequence if it affects the transcription of the sequence. In certain embodiments, the invention provides isolated nucleic acid molecules that encode an antibody of the invention, a variable light chain of the invention, and/or a variable heavy chain of the invention.

In one embodiment, a nucleic acid molecule of the invention encodes one or more VH/VL constructs as described herein. For example, a nucleic acid molecule of the invention encodes a VH/VL construct in one of the following formats: VH-A-(linker 1 or 2)-VL-B; VH-B-(linker 1 or 2)-VL-A; VH-A-(linker-1)-VL-B-(linker 3)-VH-B-(linker 2)-VL-A; VH-A-(linker 1 or 2)-VL-A; VH-B-(linker 1 or 2)-VL-B; VH-A-(linker-l)-VL-A-(linker 3)-VH-B-(linker 2)-VL-B; VL-A-(linker 1 or 2)-VH-B, VL-B-(linker 1 or 2)-VH-A; VL-A-(linker-1)-VH-B-(linker 3)-VL-B-(linker 2)-VH-A; VL-A-(linker 1 or 2)-VH-A, VL-B-(linker 1 or 2)-VH-B; VL-A-(linker-1)-VH-A-(linker 3)-VL-B-(linker 2)-VH-B; or any other orientation contemplated by those of skill in the art.

In another embodiment, the invention includes a vector comprising a nucleic acid molecule according to the invention.

The term "vector," refers to a nucleic acid molecule capable of transporting another nucleic acid to which it has been linked. One type of vector is a "plasmid," which refers to a circular double stranded DNA loop into which additional DNA segments may be ligated. Another type of vector is a viral vector, wherein additional DNA segments may be ligated into the viral genome. Certain vectors are capable of autonomous replication in a host cell into which they are introduced (*e.g.,* bacterial vectors having a bacterial origin of replication and episomal mammalian vectors). Other vectors (*e.g*., non-episomal mammalian vectors) can be integrated into the genome of a host cell upon introduction into the host cell, and thereby are replicated along with the host genome. Such expression vectors and methods of isolating expression products are generally known to those of skill in the art and are describe, for example, in Sambrook J. et al., Molecular Cloning, A Laboratory Handbook 2nd Ed., Cold Spring Harbor Laboratory Press, 1989.

The term "host cell" refers to a cell into which an expression vector has been introduced. Host cells can include bacterial, microbial, plant or animal cells. Bacteria, which are susceptible to transformation, include members of the enterobacteriaceae, such as strains of *Escherichia coli* or Salmonella; Bacillaceae, such as *Bacillus subtilis*; Pneumococcus; Streptococcus, and *Haemophilus influenzae.* Suitable microbes include *Saccharomyces cerevisiae* and *Pichia pastoris.* Suitable animal host cell lines include CHO (Chinese Hamster Ovary lines) and NSO cells.

Certain methods for preparing antibodies that bind multiple antigens are described, for example, in U.S. Patent Number 7,838,637 and U.S. Patent Number 7,129,330, the entire contents of which are expressly incorporated by reference.

Antibodies of the invention may be generated using routine techniques in the field of recombinant genetics. Knowing the sequences of the polypeptides, the cDNAs encoding them can be generated by gene synthesis by methods well known in the art. These cDNAs can be cloned into suitable vector plasmids.

It is to be understood that the antibodies of the present invention comprise the disclosed sequences rather than they consist of them. For example, cloning strategies may require that a construct is made from which an antibody with one or a few additional residues at the N-terminal end are present. Specifically, the methionine derived from the start codon may be present in the final protein in cases where it has not been cleaved post-translationally. Most constructs for scFv antibodies give rise to an additional alanine at the N-terminal end.

### Pharmaceutical Compositions

In certain embodiments, the invention provides pharmaceutical compositions comprising one or more multiple antigen-binding antibodies of the invention, together with at least one physiologically acceptable carrier or excipient. Pharmaceutical compositions may comprise, for example, one or more of water, buffers (*e.g*., neutral buffered saline or phosphate buffered saline), ethanol, mineral oil, vegetable oil, dimethylsulfoxide, carbohydrates (*e.g.,* glucose, mannose, sucrose or dextrans), mannitol, proteins, adjuvants, polypeptides or amino acids such as glycine, antioxidants, chelating agents such as EDTA or glutathione and/or preservatives.

A carrier is a substance that may be associated with an antibody prior to administration to a patient, often for the purpose of controlling stability or bioavailability of the compound. Carriers for use within such formulations are generally biocompatible, and may also be biodegradable. Carriers include, for example, monovalent or multivalent molecules such as serum albumin (*e.g*., human or bovine), egg albumin, peptides, polylysine and polysaccharides such as aminodextran and polyamidoamines. Carriers also include solid support materials such as beads and microparticles comprising, for example, polylactate polyglycolate, poly(lactide-co-glycolide), polyacrylate, latex, starch, cellulose or dextran. A carrier may bear the compounds in a variety of ways, including covalent bonding (either directly or via a linker group), noncovalent interaction or admixture.

Pharmaceutical compositions may be formulated for any appropriate manner of administration, including, for example, ocular, intranasal, otic, sublingual, transdermal, topical, oral, nasal, rectal or parenteral administration. In certain embodiments, compositions in a form suitable for oral use are preferred. Such forms include, for example, pills, tablets, troches, lozenges, aqueous or oily suspensions, dispersible powders or granules, emulsion, hard or soft capsules, or syrups or elixirs. Within yet other embodiments, compositions provided herein may be formulated as a lyophilizate. The term parenteral as used herein includes subcutaneous, intradermal, intravascular (*e.g*., intravenous), intramuscular, spinal, intracranial, intrathecal and intraperitoneal injection, as well as any similar injection or infusion technique.

In certain embodiments, an antibody of the invention can be delivered directly to the eye by ocular tissue injection such as periocular, conjunctival, subtenon, intracameral, intravitreal, intraocular, subretinal, subconjunctival, retrobulbar, or intracanalicular injections; by direct application to the eye using a catheter or other placement device such as a retinal pellet, intraocular insert, suppository or an implant comprising a porous, non-porous, or gelatinous material; by topical ocular drops or ointments; or by a slow release device in the cul-de-sac or implanted adjacent to the sclera (transscleral) or in the sclera (intrascleral) or within the eye. Intracameral injection may be through the cornea into the anterior chamber to allow the agent to reach the trabecular meshwork. Intracanalicular injection may be into the venous collector channels draining Schlemm's canal or into Schlemm's canal.

For ophthalmic delivery, an antibody of the invention may be combined with ophthalmologically acceptable preservatives, co-solvents, surfactants, viscosity enhancers, penetration enhancers, buffers, sodium chloride, or water to form an aqueous, sterile ophthalmic suspension or solution. Topical ophthalmic products may be packaged, for example, in multidose form. Preservatives may thus be required to prevent microbial contamination during use. Suitable preservatives include: chlorobutanol, methyl paraben, propyl paraben, phenylethyl alcohol, edetate disodium, sorbic acid, polyquaternium-1, or other agents known to those skilled in the art. Such preservatives are typically employed at a level of from 0.001 to 1.0% w/v. Unit dose compositions of the present invention will be sterile, but typically unpreserved. Such compositions, therefore, generally will not contain preservatives.

In certain embodiments, compositions intended to be administered topically to the eye are formulated as eye drops or eye ointments, wherein the total amount of antibody will be about 0.001 to 1.0% (w/w), preferably about 0.01 to about 1.0% (w/w).

Pharmaceutical compositions of the invention in certain circumstances will be administered as solutions for topical administration. Aqueous solutions are generally preferred, based on ease of formulation, as well as a patient's ability to easily administer such compositions by means of instilling one to two drops of the solutions in the affected eyes. However, the compositions may also be suspensions, viscous or semi-viscous gels, or other types of solid or semi-solid compositions.

Pharmaceutical compositions intended for oral use may be prepared according to any method known to the art for the manufacture of pharmaceutical compositions and may contain one or more agents, such as sweetening agents, flavoring agents, coloring agent, and preserving agents in order to provide appealing and palatable preparations. Tablets contain the active ingredient in admixture with physiologically acceptable excipients that are suitable for the manufacture of tablets. Such excipients include, for example, inert diluents (*e.g*., calcium carbonate, sodium carbonate, lactose, calcium phosphate or sodium phosphate), granulating and disintegrating agents (*e.g.,* corn starch or alginic acid), binding agents (*e.g.,* starch, gelatin or acacia) and lubricating agents (*e.g*., magnesium stearate, stearic acid or talc). The tablets may be uncoated or they may be coated by known techniques to delay disintegration and absorption in the gastrointestinal tract and thereby provide a sustained action over a longer period. For example, a time delay material such as glyceryl monosterate or glyceryl distearate may be employed.

Oily suspensions may be formulated by suspending the active ingredients in a vegetable oil (*e.g*., arachis oil, olive oil, sesame oil, or coconut oil) or in a mineral oil such as liquid paraffin. The oily suspensions may contain a thickening agent such as beeswax, hard paraffin, or cetyl alcohol. Sweetening agents, such as those set forth above, and/or flavoring agents may be added to provide palatable oral preparations. Such suspensions may be preserved by the addition of an anti-oxidant such as ascorbic acid.

Dispersible powders and granules suitable for preparation of an aqueous suspension by the addition of water provide the-active ingredient in admixture with a dispersing or wetting agent, suspending agent and one or more preservatives. Suitable dispersing or wetting agents and suspending agents are exemplified by those already mentioned above. Additional excipients, for example sweetening, flavoring and coloring agents, may also be present.

Pharmaceutical compositions may also be in the form of oil-in-water emulsions. The oily phase may be a vegetable oil (*e.g.,* olive oil or arachis oil), a mineral oil (*e.g.,* liquid paraffin), or a mixture thereof. Suitable emulsifying agents include naturally-occurring gums (*e.g.,* gum acacia or gum tragacanth), naturally-occurring phosphatides (*e.g.,* soy bean, lecithin, and esters or partial esters derived from fatty acids and hexitol), anhydrides (*e.g*., sorbitan monoleate), and condensation products of partial esters derived from fatty acids and hexitol with ethylene oxide (*e.g.,* polyoxyethylene sorbitan monoleate). An emulsion may also comprise one or more sweetening and/or flavoring agents.

The pharmaceutical composition may be prepared as a sterile injectible aqueous or oleaginous suspension in which the modulator, depending on the vehicle and concentration used, is either suspended or dissolved in the vehicle. Such a composition may be formulated according to the known art using suitable dispersing, wetting agents and/or suspending agents such as those mentioned above. Among the acceptable vehicles and solvents that may be employed are water, 1,3-butanediol, Ringer's solution and isotonic sodium chloride solution. In addition, sterile, fixed oils may be employed as a solvent or suspending medium. For this purpose any bland fixed oil may be employed, including synthetic mono- or diglycerides. In addition, fatty acids such as oleic acid may be used in the preparation of injectible compositions, and adjuvants such as local anesthetics, preservatives and/or buffering agents can be dissolved in the vehicle.

Pharmaceutical compositions may be formulated as sustained release formulations (*i.e.,* a formulation such as a capsule that affects a slow release of modulator following administration). Such formulations may generally be prepared using well known technology and administered by, for example, oral, rectal, or subcutaneous implantation, or by implantation at the desired target site. Carriers for use within such formulations are biocompatible, and may also be biodegradable; preferably the formulation provides a relatively constant level of modulator release. The amount of an antibody contained within a sustained release formulation depends upon, for example, the site of implantation, the rate and expected duration of release and the nature of the disease/disorder to be treated or prevented.

Pharmaceutical compositions provided herein are preferably administered in an amount that achieves a concentration in a body fluid (*e.g*., blood, plasma, serum, CSF, synovial fluid, lymph, cellular interstitial fluid, tears or urine) that is sufficient to detectably bind to a target molecule(s) and prevent or inhibit diseases/disorders associated with the target molecule(s). A dose is considered to be effective if it results in a discernible patient benefit.

The appropriate dosage ("therapeutically effective amount") of an antibody of the invention will depend, for example, on the condition to be treated, the severity and course of the condition, whether the antibody is administered for preventive or therapeutic purposes, previous therapy, the patient's clinical history and response to the antibody, the type of antibody used, and the discretion of the attending physician. The antibody is suitably administered to the patient at one time or over a series of treatments and may be administered to the patient at any time from diagnosis onwards. The antibody may be administered as the sole treatment or in conjunction with other drugs or therapies useful in treating the condition in question.

As a general proposition, the therapeutically effective amount of the antibody administered will be in the range of about 0.1 to about 100 mg/kg of patient body weight whether by one or more administrations, with the typical range of antibody used being about 0.3 to about 20 mg/kg, more preferably about 0.3 to about 15 mg/kg, administered daily, for example. However, other dosage regimens may be useful. The progress of this therapy is easily monitored by conventional techniques.

In another embodiment of the invention, an article of manufacture is provided comprising a container which holds an aqueous pharmaceutical formulation of a pharmaceutical composition of the invention, and optionally provides instructions for its use. Suitable containers include, for example, bottles, vials and syringes. The container may be formed from a variety of materials such as glass or plastic. An exemplary container is a 3-20 cc single use glass vial. Alternatively, for a multidose formulation, the container may be 3-100 cc glass vial. The container holds the formulation and the label on, or associated with, the container may indicate directions for use. The article of manufacture may further include other materials desirable from a commercial and user standpoint, including other buffers, diluents, filters, needles, syringes, and package inserts with instructions for use.

In certain embodiments, the invention provides:
1. A multiple antigen-binding antibody molecule comprising:
   a) a heavy chain variable domain with a specificity for antigen A (VH-A) linked to a light chain variable domain with a specificity for antigen B (VL-B) by peptide linker 1 to form a VH-A/VL-B construct;
   b) a heavy chain variable domain with a specificity for antigen B (VH-B) linked to a light chain variable domain with a specificity for antigen A (VL-A) by peptide linker 2 to form a VH-B/VL-A construct;
   wherein the multiple antigen-binding antibody lacks constant domains, at least one of VL-A and VL-B comprises an Arginine at AHo position 50, and/or at least one of VH-A and VH-B comprises at least one of the following: a Serine at AHo position 12, a Serine or Threonine at AHo position 103, and a Serine or Threonine at AHo position 144.
2. The multiple antigen-binding antibody of 1, wherein at least one of VL-A and VL-B comprises a framework sequence that has at least 65% identity to the sequence of SEQ ID NO: 5.
3. The multiple antigen-binding antibody of 1, wherein at least one of VH-A and VH-B comprises a framework sequence that has at least 80% identity to the sequence of SEQ ID NO: 6.
4. The multiple antigen-binding antibody of 1, wherein at least one of the VL-A/VH-B and the VH-B/VL-A constructs comprises a human Vkappa1 family light chain variable region, a human Vlambda 1 family light chain variable region, or a human Vkappa3 family light chain variable region.
5. The multiple antigen-binding antibody of 1, wherein at least one of the VL-A/H-B and the VH-B/VL-A constructs comprises a human VH3 family heavy chain variable region, a human VH1a family heavy chain variable region, or a human VH1b family heavy chain variable region.
6. The multiple antigen-binding antibody of 1, wherein the VH domains and the VL domains comprise CDRs from a lagomorph.
7. The multiple antigen-binding antibody of 1, wherein VH-A and/or VH-B comprise Serine at AHo position 12, Threonine at AHo position 103, and Threonine at AHo position 144.
8. The multiple antigen-binding antibody of 1, wherein the Arginine at AHo position 50 of VL-A and/or VL-B is introduced by substitution.
9. The multiple antigen-binding antibody of 1, wherein at least one of Serine at AHo position 12, Serine or Threonine at AHo position 103, and Serine or Threonine at AHo position 144 of VH-A and/or VH-B are introduced by substitution.
10. The multiple antigen-binding antibody of 1, wherein peptide linker 1 and peptide linker 2 each have 1-10 amino acids.
11. The multiple antigen-binding antibody of 1, further comprising peptide linker 3 which links the VH-A/VL-B construct to the VH-B/VL-A construct, the peptide linker 3 having 10-30 amino acids.
12. The multiple antigen-binding antibody of 11, wherein peptide linker 1 and peptide linker 2 each have 3-7 amino acids, and peptide linker 3 has 15-20 amino acids.
13. The multiple antigen-binding antibody of 12, wherein peptide linker 1 and peptide linker 2 each have 5 amino acids, and peptide linker 3 has 20 amino acids.
14. The multiple antigen-binding antibody of 13, wherein peptide linker 1 comprises the sequence of GGGGS (SEQ ID NO: 1), peptide linker 2 comprises the sequence of GGGGS (SEQ ID NO: 1), and peptide linker 3 comprises the sequence of (GGGGS)₄ (SEQ ID NO: 4).
15. The multiple antigen-binding antibody of 14, having the format VH-A-SEQ ID NO: 1-VL-B-SEQ ID NO: 4-VH-B-SEQ ID NO: 1-VL-A.
16. The multiple antigen-binding antibody of 1, wherein the antibody is bivalent.
17. The multiple antigen-binding antibody of 1, wherein the antibody is bispecific.
18. A pharmaceutical composition comprising the multiple antigen-binding antibody of 1.
19. Use of the multiple antigen-binding antibody of 1 for diagnosis and/or treatment of a disease.
20. A nucleic acid molecule encoding the VH-A/VL-B construct and/or VH-B/VL-A construct of 1.
21. A vector comprising the nucleic acid molecule of 20.
22. An isolated host cell comprising the vector of 21.
23. A nucleic acid molecule encoding the antibody of 11.
24. A vector comprising the nucleic acid molecule of 23.
25. An isolated host cell comprising the vector of 24.
26. A multiple antigen-binding antibody comprising:
   a) a single-chain antibody comprising a heavy chain variable domain with a specificity for antigen A (VH-A) linked to a light chain variable domain with a specificity for antigen A (VL-A) by peptide linker 3 to form scFv-A;
   b) a single-chain antibody comprising a heavy chain variable domain with a specificity for antigen B (VH-B) linked to a light chain variable domain with a specificity for antigen B (VL-B) by peptide linker 3 to form scFv-B;
   wherein scFv-A is linked to scFv-B by a peptide linker 1, and at least one of VL-A and VL-B comprises an Arginine at AHo position 50, and at least one of VH-A and VH-B comprises at least one of the following: Serine at AHo position 12, a Serine or Threonine at AHo position 103, and a Serine or Threonine at AHo position 144.
27. The multiple antigen-binding antibody of 26, wherein VH-A and/or VH-B comprise Serine at AHo position 12, Threonine at AHo position 103, and Threonine at AHo position 144.
28. The multiple antigen-binding antibody of 26, wherein the Arginine at AHo position 50 of VL-A and/or VL-B is introduced by substitution.
29. The multiple antigen-binding antibody of 26, wherein at least one of Serine at AHo position 12, Serine or Threonine at AHo position 103, and Serine or Threonine at AHo position 144 of VH-A and/or VH-B is introduced by substitution.
30. The multiple antigen-binding antibody of 26, wherein peptide linker 1 has 1-10 amino acids.
31. The multiple antigen-binding antibody of 26, wherein peptide linker 3 has 10-30 amino acids.
32. The multiple antigen-binding antibody of 26, wherein peptide linker 1 has 3-7 amino acids, and peptide linker 3 has 15-20 amino acids.
33. The multiple antigen-binding antibody of 26, wherein peptide linker 1 has 5 amino acids, and peptide linker 3 has 15 amino acids.
34. The multiple antigen-binding antibody of 26, wherein at least one of VL-A or VL-B comprises a framework sequence that has at least 65% identity to the sequence of SEQ ID NO: 6.
35. The multiple antigen-binding antibody of 26, wherein at least one of VH-A or VH-B comprises a framework sequence that has at least 80% identity to the sequence of SEQ ID NO: 7.
36. The multiple antigen-binding antibody of 26, wherein at least one of scFv-A and scFv-B comprises a human Vkappa1 family light chain variable region, a human Vlambda 1 family light chain variable region, or a human Vkappa3 family light chain variable region.
37. The multiple antigen-binding antibody of 26, wherein at least one of scFv-A and scFv-B comprises a human VH3 family heavy chain variable region, a human VHla family heavy chain variable region, or a human VH1b family heavy chain variable region.
38. The multiple antigen-binding antibody of 26, wherein the VH domains and the VL domains comprise CDRs from a lagomorph.
39. A multiple antigen-binding antibody molecule comprising:
   a) CDRs from a lagomorph;
   b) a heavy chain variable domain with a specificity for antigen A (VH-A) linked to a light chain variable domain with a specificity for antigen B (VL-B) by peptide linker 1 to form a VH-A/VL-B construct;
   c) a heavy chain variable domain with a specificity for antigen B (VH-B) linked to a light chain variable domain with a specificity for antigen A (VL-A) by peptide linker 2 to form a VH-B/VL-A construct; and
   d) a peptide linker 3 having 10-30 amino acids linking a VH-A/VL-B construct to a VH-B/VL-A construct;
   wherein at least one of the heavy chain variable domains comprises at least three of the following: threonine (T) at AHo position 24, valine (V) at AHo position 25, alanine (A) or glycine (G) at AHo position 56, lysine (K) at AHo position 82, threonine (T) at AHo position 84, valine (V) at AHo position 89 and arginine (R) at AHo position 108, and peptide linker 1 and peptide linker 2 each have 1-10 amino acids.
40. The multiple antigen-binding antibody of 39, wherein at least one of VL-A and VL-B comprises a framework sequence that has at least 85% identity to the sequence of SEQ ID NO: 5.
41. The multiple antigen-binding antibody of 39, wherein at least one of VH-A and VH-B comprises a framework sequence that has at least 90% identity to the sequence of SEQ ID NO: 6.
42. The multiple antigen-binding antibody of 39, further comprising glutamic acid (E) at AHo position 1, valine (V) at AHo position 3, leucine (L) at AHo position 4, Serine (S) at AHo position 10; Arginine (R) at AHo position 47, Serine (S) at AHo position 57, phenylalanine (F) at AHo position 91 and/or Valine (V) at AHo position 103 in at least one of the variable light chain domains.
43. The multiple antigen-binding antibody of 39, wherein at least one of the heavy chain variable domains comprises at least one of the following: a Serine at AHo position 12, a Serine or Threonine at AHo position 103, and a Serine or Threonine at AHo position 144.
44. The multiple antigen-binding antibody of 43, wherein at least one of Serine at AHo position 12, Serine or Threonine at AHo position 103, and Serine or Threonine at AHo position 144 of VH-A and/or VH-B are introduced by substitution.
45. The multiple antigen-binding antibody of 39, wherein peptide linker 1 and peptide linker 2 each have 5 amino acids.
46. The multiple antigen-binding antibody of 45, wherein peptide linker 3 has 15-20 amino acids.
47. The multiple antigen-binding antibody of 46, wherein peptide linker 1 comprises the sequence of GGGGS (SEQ ID NO: 1), peptide linker 2 comprises the sequence of GGGGS (SEQ ID NO: 1), and peptide linker 3 comprises the sequence of (GGGGS)₄ (SEQ ID NO: 4).
48. The multiple antigen-binding antibody of 47, having the format VH-A-SEQ ID NO: 1-VL-B-SEQ ID NO: 4-VH-B-SEQ ID NO: 1-VL-A.
49. The multiple antigen-binding antibody of 39, wherein the antibody is bivalent.
50. The multiple antigen-binding antibody of 39, wherein the antibody is bispecific.
51. A pharmaceutical composition comprising the multiple antigen-binding antibody of 39.
52. Use of the multiple antigen-binding antibody of 39 for diagnosis and/or treatment of a disease.
53. A nucleic acid molecule encoding the VH-A/VL-B construct and/or VH-B/VL-A construct of 39.
54. A vector comprising the nucleic acid molecule of 53.
55. An isolated host cell comprising the vector of 54.

The contents of any patents, patent applications, and references cited throughout this specification are hereby incorporated by reference in their entireties.

Unless otherwise required by context, singular terms used herein shall include pluralities and plural terms shall include the singular.

### EXAMPLES

The present disclosure is further illustrated by the following examples, which should not be construed as further limiting. The contents of all figures and all references, patents and published patent applications cited throughout this application are expressly incorporated herein by reference in their entireties.

### Example 1

### Molecular design, cloning and expression of bispecific and bivalent antibodies derived from rabbit antibodies.

DNA sequences encoding bispecific and bivalent antibodies were generated by oligonucleotide synthesis from digital genetic sequences and subsequent annealing of the resulting fragments using overlap extension techniques. All sequences were optimized for E. coli codon usage, GC content, mRNA secondary structure, codon and motif repeats, and restriction sites. Different amino acid linkers, see Table 1, were used to connect humanized VL and VH domains from rabbit antibodies with different specificities.

**Table 1**

| Linker 1 and 2 | |
|---|---|
| 3aa | GGS |
| 5aa | GGGGS (SEQ ID NO: 1) |
| 7aa | GGGGGGS (SEQ ID NO: 2) |

| Linker 3 | |
|---|---|
| 15aa | GSDSNAGRASAGNTS (SEQ ID NO: 3) |
| 20aa | (GGGGS)₄ (SEQ ID NO: 4) |

Multiple antigen binding-antibody molecules were prepared to be bispecifically and bivalently binding to target molecules. All molecules were cloned in expression vectors for insoluble E.Coli expression, which contain a T7lac promoter, a bacterial ribosome binding site followed by the antibody molecule. E. coli BL21(DE3) transformed with the respective inclusion body expression plasmids were grown at 37°C in dYT medium containing the appropriate antibiotics. Protein expression was initiated by addition of 1 mM isopropyl 1-thio-D-galactopyranoside (final concentration) at an absorbance (A600) of about 2.0. Three hours after induction, E. coli cells were harvested and disrupted by sonication, and inclusion bodies were isolated by repeated washing and centrifugation steps.

Inclusion bodies were solubilized at a concentration of 10 mg/ml in the presence of 6 M Gdn-HCl and reduced by addition of 20 mM dithiothreitol. Basic refolding screenings were performed to select best pH, redox system (cystine/cysteine), and salt concentrations from the range of tested conditions. Best conditions for each individual antibody were used for a lab-scale refolding process. The bispecific or bivalent antibody proteins were re-natured by rapid dilution into a 50-fold volume of refolding buffer. After up-concentration and dialysis against PBS buffer, pH 6.0, proteins were purified using size-exclusion chromatography.

### Example 2

### Generation of bivalent antibodies

Bivalent antibodies binding to interleukin 23 (IL-23) were generated based on the variable domains of the rFW1.4 framework, a human scaffold for generic grafting of rabbit antibodies (as disclosed in International Application No. WO 2009/155726), and which is essentially compatible with all rabbit antibodies. Three formats were produced, including diabodies, single-chain diabodies, and tandem single-chain antibodies. CDRs were taken from an antibody that was shown to bind human IL-23. Diabodies (Db) were obtained by expression of two fragments of the format VHA-Linker 1-VLB and VHB-Linker 2-VLA in the same cell resulting in the formation of heterodimers, each one of the coding sequences being preceded by a ribosome binding site (RBS). In these molecules, linker 1 and 2 were 5 amino acids (GGGGS, SEQ ID NO: 1). In another format, the two polypeptide chains were fused by an additional middle linker (linker 3), generating a single gene encoding a single-chain diabody (scDb): VHA-Linker 1-VLB-Linker3-VHB-Linker2-VLA, where linker 3 consisted of 15 amino acids (GSDSNAGRASAGNTS, SEQ ID NO: 3) (Völkel et al., 2001, Protein Eng 14:815-823). A third format, tandem scFv (TdscFv) was produced by connecting two scFvs through a short middle linker (GGGGS, SEQ ID NO: 1) and a long linker 3 ((GGGGS)₄, SEQ ID NO: 4, resulting in a domain order of VL-A-Linker 3-VH-A-Linker 1-VL-B-Linker3-VH-B, to generate a bivalent molecule in which VH-A and VH-B were identical, as well as VL-A and VL-B.

The effects of different features on the framework regions, in the scDb format, were evaluated for producibility, stability and tendency to form oligomers. The formats are described in Table 2. Briefly, molecule #1 consisted of variable domains of the rFW1.4 in a scDb with no additional substitutions. Molecule #2 was a variant of #1 where Arginine at AHo position 50 was introduced on both VL domains (VL-A/-B). Molecule #3 was also based on #1, but had three substitutions that were introduced on both VH domains (VH-A/- B), specifically serine at AHo position 12, Threonine at AHo position 103, and Threonine at AHo position 144. Molecule #14 consisted of molecule #1 with the substitutions of the Arginine at AHo position 50 on both VL domains (VL- A/-B), and Serine at AHo position 12, Threonine at AHo position 103, and the Threonine at AHo position 144 on both VH domains (VH-A/-B). For comparison purposes an additional scDb (#5) was generated using the consensus sequences of the human germline antibody repertoire (Knappik et al., 2000, J. Mol. Biol. 296:57-86). The framework regions correspond to the consensus sequence of the VH3 and VL kappa 1 subtypes, designated HuCal. This humanized scDb was generated with the same CDRs used in the other bivalent scDbs described herein, thus differences are located only at the framework regions.

**Table 2**

| | |
|---|---|
| #1 | rFW 1.4 |
| #2 | rFW1.4, VL-A/-B: arginine at AHo position 50 |
| #3 | rFW 1.4,VH-A/-B: serine at AHo position 12, threonine at AHo position 103, threonine at AHo position 144 |
| #4 | rFW1.4, VL-A/-B: arginine at AHo position 50, serine at AHo position 12, threonine at AHo position 103, threonine at AHo position 144 |
| #5 | HuCal FW graft of CDRs |

### Example 3

### Generation of bispecific antibodies

Bispecific single-chain diabodies were designed to engage in one single molecule two different specificities. The VH and VL domains from two different scFv antibodies originally generated by humanization of rabbit antibodies against VEGF₁₆₅ and TNFα were used as source of the variable region genes to construct a single fragment of the format VHA-Linkerl-VLB -linker3-VHB-Linker2-VLA, where variable domains labeled with A bind VEGF165 and the ones labelled with B bind TNFα. The antibody binding VEGF₁₆₅ had a VL sequence of: 
EIVMTQSPSTLSASVGDRVIITCQASEIIHSWLAWYQQKPGKAPKLLIYLASTLASGVPSRFSGSGSGAEFTLTI

SSLQPDDFATYYCQNVYLASTNGANFGQGTKLTVLG, SEQ ID NO: 7;
 and a VH having the sequence of:

EVQLVESGGGLVQPGGSLRLSCTASGFSLTDYYYMTWVRQAPGKGLEWVGFIDPDDDPYYATWAKGRFTISRDNS
KNTLYLQMNSLRAEDTAVYYCAGGDHNSGWGLDIWGQGTLVTVSS, SEQ ID NO: 8.

The sequence of the VH domain having the serine at AHo position 12, threonine at AHo position 103, and threonine at AHo position 144 was:

EVQLVESGGGSVQPGGSLRLSCTASGFSLTDYYYMTWVRQAPGKGLEWVGFIDPDDDPYYATWAKGRFTISRDNS
KNTLYLQMNSLRAEDTATYYCAGGDHNSGWGLDIWGQGTTVTVSS, SEQ ID NO: 9.

The antibody binding TNFα had a VL sequence of: 
EIVMTQSPSTLSASVGDRVIITCQSSQSVYGNIWMAWYQQKPGRAPKLLIYQASKLASGVPSRFSGSGSGAEFTL
TISSLQPDDFATYYCQGNFNTGDRYAFGQGTKLTVLG, SEQ ID NO: 10;
 and a VH having the sequence of:

EVQLVESGGGSVQPGGSLRLSCTASGFTISRSYWICWVRQAPGKGLEWVGCIYGDNDITPLYANWAKG
RFTISRDTSKNTVYLQMNSLRAEDTATYYCARLGYADYAYDLWGQGTTVTVSS, SEQ ID
NO: 11.

The bispecific antibodies were designed and constructed using the standard DNA manipulation techniques described in Example 1. The effects of different framework features introduced at framework regions as well as different linker combinations were assessed on different bispecific scDb, described in Table 3.

**Table 3**

| | |
|---|---|
| #6 | FW1.4, linker (5aa-15aa-5aa) |
| #7 | rFW1.4, linker (5aa-15aa-5aa) |
| #8 | rFW1.4, linker (5aa-15aa-5aa), VL-A: arginine at AHo position 50 |
| #9 | rFW1.4, linker (5aa-15aa-5aa), VL-A: arginine at AHo position 50, VH-B: serine at AHo position 12, threonine at AHo position 103, threonine at AHo position 144 |
| #10 | rFW1.4, linker (5aa-15aa-5aa), VL-A: arginine at AHo position 50, VH-A/-B: serine at AHo position 12, threonine at AHo position 103, threonine at AHo position 144 |
| #11 | rFW1.4, linker (5aa-20aa-5aa), VL-A: arginine at AHo position 50, VH-A/-B: serine at AHo position 12, threonine at AHo position 103, threonine at AHo position 144 |
| #12 | rFW1.4, linker (7aa-20aa-7aa), VL-A: arginine at AHo position 50, VH-A/-B: serine at AHo position 12, threonine at AHo position 103, threonine at AHo position 144 |
| #13 | rFW1.4, linker (3aa-20aa-3aa), VL-A: arginine at AHo position 50, VH-A/-B: serine at AHo position 12, threonine at AHo position 103, threonine at AHo position 144 |

The sequence of the expressed construct having the 5-20-5 linker combination and serine at AHo position 12, threonine at AHo position 103, and threonine at AHo position 144 was:

MEVQLVESGGGSVQPGGSLRLSCTASGFSLTDYYYMTWVRQAPGKGLEWVGFIDPDDDPYYATWAKGRFTISRDN
SKNTLYLQMNSLRAEDTATYYCAGGDHNSGWGLDIWGQGTTVTVSSGGGGSEIVMTQSPSTLSASVGDRVIITCQ
SSQSVYGNIWMAWYQQKPGRAPKLLIYQASKLASGVPSRFSGSGSGAEFTLTISSLQPDDFATYYCQGNFNTGDR
YAFGQGTKLTVLGGGGGSGGGGSGGGGSGGGGSEVQLVESGGGSVQPGGSLRLSCTASGFTISRSYWICWVRQAP
GKGLEWVGCIYGDNDITPLYANWAKGRFTISRDTSKNTVYLQMNSLRAEDTATYYCARLGYADYAYDLWGQGTTV
TVSSGGGGSEIVMTQSPSTLSASVGDRVIITCQASEIIHSWLAWYQQKPGKAPKLLIYLASTLASGVPSRFSGSG
SGAEFTLTISSLQPDDFATYYCQNVYLASTNGANFGQGTKLTVLG     (SEQ ID NO: 12).

### Example 4

### Characterization of bispecific and bivalent antibodies.

### Producibility

Insoluble expressed proteins were refolded and purified by preparative Size-exclusion high-performance liquid chromatography (SE-HPLC). Resulting protein was characterised according to its purified protein yield, in mg per liter culture media. This value gave a characteristic measurment of the producibility of the respective molecule. Purity was defined as the monomer content, excluding soluble aggregates, of samples after purification of the refolded proteins. The purity was determined by a preparative size-exclusion chromatography. Peaks of monomers and soluble aggregates were resolved from non-monomeric species using a TSKgel Super SW2000 column (TOSOH Bioscience). The percentage of monomeric protein was calculated as the area of the monomer peak divided by the total area of all product peaks.

### Thermostability measurements (FT-IR, DSC)

The molecules were concentrated up to 3mg/ml and the flow through was collected for the blank measurement. FT-IR (Fourir Transfom-Infrared Spectroscopy) reading and DSC (Capillary Differential Scanning Calorimetry) were performed to measure thermal stability. FT-IR spectra were obtained by using the FT-IR Bio-ATR (attenuated total reflection) cell in a Tensor Bruker machine. The denaturation profiles, showing changes in secondary structure, were obtained by thermo challenging the molecules with a temperature gradient in 5°C steps (25°C to 95°C). All spectra manipulations were performed using OPUS software. Normalization was performed against the transient atmospheric (CO₂ and H₂O) background and the blank samples. The resulting protein spectrum was then baseline corrected and the protein amide I spectra was determined from the width of the widest resolvable peak in the expected region. Second derivative spectra were obtained for the amide I band spectra using a third degree polynomial function with a smoothing function. Changes in protein structure were estimated by amide I second derivative analysis using a linear calibration curve for the initial curve-fit calculations assuming 0% denatured protein for the 3 low temperature measurements and 100% denatured protein for the 3 high temperature measurements. The denaturation profiles were used to approximate midpoints of the thermal unfolding transitions (Tm) for every variation applying a Boltzmann sigmoidal model. DSC measurement also thermally unfolded the samples. The differential scanning calorimeter (MicroCal capillary VP-DSC) used a temperature gradient of 200°C/h. Data analysis was performed by doing a reference reduction of the buffer signal and normalization to the respective protein concentration in µM with a subsequent baseline correction, all manipulations were performed in the MicroCal software of the DSC. The Tm was the temperature equaling the point when most of the energy uptake occurred, which represented the temperature of unfolding.

### Short Term Stability test

Protein was examined before and after two weeks of incubation at 40°C, for soluble aggregates and degradation products. Proteins were concentrated to the following desired concentrations: 10mg/ml, 20mg/ml, 40mg/ml, and 60mg/ml. In the case the protein was not soluble enough to reach the desired concentrations, the highest possible concentration was analysed. The highest concentration was reached when further concentrating only led to precipitation without increase of concentration. These samples were analysed on day 0 and day 14. Analysis of purity and possibly appearing degradation bands was done at both time points by 12.5% sodium dodecyl sulfate-polyacrylamide gel electrophoresis (SDS-PAGE). Soluble oligomerations and aggregates were assessed by size exclusion high-performance liquid chromatography (SE)-HPLC, before and after the incubation period. Monomers were resolved from non-monomeric species on a TSKgel Super SW2000 column (TOSOH Bioscience) and the percentage of monomeric protein was calculated as the area of the monomer peak divided by the total area of all product peaks. Total concentration was determined by UV absorption measurement at wavelength 280nm using nanodrop device. In this way, this test of short term stability assessed properties like solubility, stability, aggregation and oligomerization.

### Results

### Bivalent molecules binding IL23:

Different scFv like formats, bivalently binding IL-23, were tested. These molecules comprised the VH and VL domains binding IL-23 as described in Example 2, above. All molecules were characterized according to their production properties, thermal stability and short term stability as described in methods.

The tested formats in particular included:
Molecule #14: Diabody (Db): VHA-Linker 1-VLB and VHB-Linker 2-VLA; (linker 1 and 2 = SEQ ID NO: 1);
Molecule #15: single chain Diabody (scDb): VHA-Linker 1-VLB-Linker3-VHB-Linker2-VLA; (linker 1 and 2 = SEQ ID NO: 1; linker 3 = SEQ ID NO: 4);
Molecule #16: Tandem scFv (TdscFv): VLA-Linker 3-VHA-Linker 1-VLB-Linker3-VHB; (linker 3 = SEQ ID NO: 4; linker 1 = SEQ ID NO: 1).

In all these formats VL -A and -B were identical, and the VH-A and -B were identical, thus producing a bivalent antibody binding to IL-23.

Producibility of scDb and TdscFv antibodies in bacterial systems is often limited by their relatively low yields and their tendency to form aggregates. However, all the formats evaluated were efficiently produced by refolding from purified inclusion bodies. Refolded proteins were mainly monomeric after subsequently purification by preparative size-exclusion chromatography, see Table 4. The TdscFv #16, showed the highest yield but the lowest purity of 89%. The Db #15 and scDb #14 had similar yield, and the scDb showed the highest purity measured as monomer content by SE-HPLC.

**Table 4**

| **#** | **Yield [mg/L]** | **SE-HPLC purity [%]** |
|---|---|---|
| #14 | 83.5 | 98 |
| #15 | 88 | 92 |
| #16 | 158 | 89 |

All three formats showed a similar Tm, of approximately 73°C, when measured with the Differential Scanning Calorimetry (DSC). Results are shown in table 5.

**Table 5**

| **#** | **Tm app. [°C]** |
|---|---|
| #14 | 73.8 |
| #15 | 73.1 |
| #16 | 73.2 |

Subtle differences in Tm measured by FT-IR were observed, see Table 6. In agreement with DSC measurements these results confirmed all three formats are thermally stable.

**Table 6**

| **#** | **Tm [°C]** |
|---|---|
| #14 | 70.3 |
| #15 | 68.9 |
| #16 | 67.1 |

Solubility and stability were tested in a short term stability test, when samples are incubated at 40°C for a 14 day period, see Table 7. The Db #15 and the scDb #14 were only soluble to a concentration up to approximately 20mg/ml, but they still stayed on high monomer content of approximately 95%, on day 14. This reflected a high stability in this short time period. The TdscFv #16 was soluble up to 40mg/ml, but lost monomer content during the 14 day period, resulting into 66.6% monomer content. Hence, this experiment demonstrated that scDb #14 and Db #15 had reduced propensitiy for aggregation compared with TdscFv #16 under these conditions.

**Table 7**

| **#** | **SE-HPLC purity [%]** | **Conc. [mg/ml]** |
|---|---|---|
| #14 | 95.6 | 23 |
| #15 | 93.3 | 26 |
| #16 | 66.1 | 40 |

### Bivalent molecules binding IL23, scDb format with framework features:

Different scDb variants described in Table 2, bivalently binding IL-23, were then tested. All molecules were characterized according to their production properties, thermal stability and short term stability as described in the methods.

All bivalent scDb molecules were produced in this domain order: VHA -Linker1-VLB-Linker3-VHB-linker2-VLA (linker 1 and 2 = SEQ ID NO: 1; and linker 3 = SEQ ID NO: 4), whereas VL-A and -B were identical, and VH-A and -B were identical, with the specific substitutions summarized in Example 2, Table 2.

All bivalent molecules based on the rFW1.4 framework were well producible, and samples were mainly monomeric after purification by preparative size-exclusion chromatography, see Table 8. The scDb #5 based on the germline consensus framework showed the lowest production yield and monomer content of the purified sample.

**Table 8**

| **#** | **Yield [mg/L]** | **SE-HPLC purity [%]** |
|---|---|---|
| **#5** | 19 | 61.6 |
| **#1** | 65 | 98 |
| **#3** | 65 | 99 |
| **#2** | 101 | 98 |
| **#4** | 40 | 98 |

All bivalent molecules based on the rFW1.4 framework showed high Tm, of approximately 73°C, in Differential Scanning Calorimetry (DSC). The scDb based on the germline consensus framework showed the lowest Tm, of 66°C, see Table 9.

**Table 9**

| **#** | **Tm app. [°C]** |
|---|---|
| **#5** | 66 |
| **#1** | 74.5 |
| **#3** | 73.6 |
| **#2** | 73.2 |
| **#4** | 73.4 |

Clear differences in stability and maximal reached concentration were observed between the different versions of bivalent IL-23 binding molecules, see Table 10. The scDb #5 based on the germline consensus showed a decreased monomer content of 44%, after two weeks incubation at 40°C. Molecules based on the rFW1.4 framework remained mainly monomeric after the two weeks incubation at 40°C, and showed monomer contents from 87-95%.

The variant #2, in which arginine was introduced at AHo residue position 50 on both VL domains, showed the highest monomer content after 2 weeks incubation at 40°C compared to the #1, which had a Lysine in both VL domains at residue position 50. Also, variant #3, which contained Serine at AHo position 12, the Threonine at AHo position 103, and the Threonine at AHo position 144 on both VH domains, showed higher monomer content, compared to #1 after two weeks incubation at 40°C. Also, the combination of these substitutions, scDb #4, resulted in an increased ability to concentrate the protein, which meant a further increase of solubility, see Table 10.

**Table 10**

| **#** | **SE-HPLC purity [%]** | **Conc. [mg/ml]** |
|---|---|---|
| **#5** | 44.4 | 2 |
| **#1** | 87.5 | 2.1 |
| **#3** | 95.7 | 1.9 |
| **#2** | 90.1 | 35 |
| **#4** | 94.9 | 41 |

### Bispecific molecules binding VEGF and TNFα:

Different scDb variants bispecifically binding VEGF and TNFα were tested. See Example 3, Table 3 for the differences between the molecules. All molecules were characterized according to their production properties, thermal stability and short term stability.

All bivalent scDb molecules were in this order: VHA-linker 1-VLB-Linker3-VHB-linker2-VL-A. Domains VL-A and VH-A assembled the TNFα binding antibody fragment. Domains VL-B and VH-B assembled the VEGF binding antibody fragment. The introduced substitutions and changes in linker sequence are summarized specifically in Example 3, Table 3.

All bispecific molecules were producible, but reached different yields and monomer contents, see Table 11. Comparing #11 scDb version, which contained linker 3 consisting of 20 aa (SEQ ID NO: 4) to version #9, which contained the same substitutions but only differed by the linker 3 (being SEQ ID NO: 3). ScDb version #11 showed increased yield and purity, of 61mg/ml with 75%, whereas, scDb #9 only had a yield of 7mg/ml with a purity of 34%. Additional substitutions on #10 increased purity significantly, when compared to the scDb version without any substitutions #6.

**Table 11**

| **#** | **Yield [mg/L**] | **SE-HPLC purity [%]** |
|---|---|---|
| **#6** | 13 | 19 |
| **#7** | 6 | 72 |
| **#8** | 25 | 71 |
| **#9** | 7 | 34 |
| **#10** | 4 | 96 |
| **#11** | 62 | 75 |
| **#12** | 34 | 74 |
| **#13** | 11 | 69 |

All bispecific scDb molecules with substitutions show a high Tm, of higher than 72°C, in DSC measurement for thermal stability. Especially, when compared to #6, the version without any substitutions, which only reached a Tm of 57.2°C, see Table 12. Exchange of linker structures did not change the thermal stability. These scDb variations, #11, #12, and #13 also still had a Tm of 74°C.

**Table 12**

| **#** | **Tm app. [°C]** |
|---|---|
| **#6** | 57.2 |
| **#7** | 72.1 |
| **#8** | 72.5 |
| **#9** | 74.2 |
| **#10** | 74.8 |
| **#11** | 74.8 |
| **#12** | 74.6 |
| **#13** | 75.4 |

The bispecific scDb variants, #11, #12, and #13, which had a linker 3 consisting of 20aa (SEQ ID NO: 4), showed a Tm of approximately 69°C, when measured by in FT-IR. These values were compared to the Tm of #9, of 66.2°C, which contained the 15aa linker 3 (SEQ ID NO: 3). These results of thermal stability showed that exchanging the linker 3, from 15aa to 20aa increased the Tm of the scDb molecule, as shown in Table 13.

**Table 13**

| **#** | **Tm app. [°C]** |
|---|---|
| **#9** | 66.2 |
| **#11** | 69.8 |
| **#12** | 68.9 |
| **#13** | 69.5 |

Clear differences in solubility and stability were observed during the concentrating process and after incubation for 14 day on 40°C for the bispecific scDb versions. The substitutions introduced on the FW regions increased solubility and stability of the scDb proteins, see Table 14. Version #10, which had additional substitutions on VH-B, had a monomer content of 88%, on day 14. These values are compared to #9 with 53% monomer content and #6 with 19% monomer content, on day 14. These results showed that the stability was enhanced by the substitutions. Molecules were excluded from this comparison, when purity was higher on day 14 than on day 0.

Exchanging the linker 3 from the 15aa sequence (SEQ ID NO: 3) to 20aa (SEQ ID NO: 4) led to an increased solubility and stability of the scDb molecule, see Table 14. Differences in solubility and stability of molecules #9 compared with #11, #12, and #13 support this conclusion. Variants of scDb, #9 and #11, #12, and #13 contained the same substitutions, but different linker 3 sequences, see Example 3, Table 3. #11 version of scDb with linker 3, 20aa (SEQ ID NO: 4), and linker 1 and 2, 5aa (SEQ ID NO: 1), showed a monomer content of 81% at a concentration of 40mg/ml, whereas, #9 only reached 10mg/ml with a monomer content of 53%, see Table 14.

**Table 14**

| **#** | **SE-HPLC purity [%]** | **Conc. [mg/ml]** | |
|---|---|---|---|
| **#6** | 19.3 | 5.5 | |
| **#7** | 99.1 | 1.5 | * |
| **#8** | 92.5 | 8.7 | * |
| **#9** | 53.2 | 10 | * |
| **#10** | 88.8 | 1.5 | + |
| **#11** | 81.2 | 40 | |
| **#12** | 83.7 | 40 | |
| **#13** | 43.0 | 40 | |

It should be understood that the foregoing disclosure emphasizes certain specific embodiments of the invention and that all modifications or alternatives equivalent thereto are within the spirit and scope of the invention as set forth in the appended claims.

### Further aspects and embodiments of the invention include:

1. A multiple antigen-binding antibody molecule comprising:
   a) two heavy chain variable domains, one with specificity to antigen A (VH-A) and one with specificity for antigen B (VH-B), and
   b) two light chain variable domains, one with specificity to antigen A (VL-A) and one with specificity for antigen B (VL-B),
   wherein at least one of the two heavy chain variable domains comprises at least one of the following: a Serine at AHo position 12, a Serine or Threonine at AHo position 103, and a Serine or Threonine at AHo position 144; and/or
   wherein at least one of the two light chain variable domains comprises an Arginine at AHo position 50.
2. The multiple antigen-binding antibody of item 1, wherein VH-A is linked to VL-B by peptide linker 1 to form a VH-A/VL-B construct and VH-B is linked to VL-A by peptide linker 2 to form a VH-B/VL-A construct.
3. The multiple antigen-binding antibody of item 2, wherein the VH-A/VL-B construct is in a VH-A-(linker 1)-VL-B orientation.
4. The multiple antigen-binding antibody of item 2, wherein the VH-B/VL-A construct is in a VH-B-(linker 2)-VL-A orientation.
5. The multiple antigen-binding antibody of item 2, wherein peptide linker 1 and peptide linker 2 each have 1-10 amino acids.
6. The multiple antigen-binding antibody of item 5, wherein peptide linker 1 has the sequence of GGGGS (SEQ ID NO: 1) and peptide linker 2 has the sequence of GGGGS (SEQ ID NO: 1).
7. The multiple antigen-binding antibody of item 2, wherein VH-A/VL-B construct is further linked to VH-B/VL-A construct by peptide linker 3.
8. The multiple antigen-binding antibody of item 7, wherein peptide linker 3 has 10-30 amino acids.
9. The multiple antigen-binding antibody of item 8, wherein peptide linker 3 has the sequence of (GGGGS)₄ (SEQ ID NO: 4).
10. The multiple antigen-binding antibody of item 1, having the format VH-A-SEQ ID NO: 1-VL-B-SEQ ID NO: 4-VH-B-SEQ ID NO: 1-VL-A.
11. The multiple antigen-binding antibody of item 1, wherein the VH-A is linked to VL-A to form a single chain antibody with specificity for antigen A (scFv A) and VH-B is linked to VL-B to form a single chain antibody with specificity for antigen B (scFv B).
12. The multiple antigen-binding antibody of item 11, wherein the scFv-A is linked to the scFv-B in the following format: VH-A/VL-A - linker 3 - VH-B/VL-B.
13. The multiple antigen-binding antibody of item 12, wherein the linker 3 has the sequence of SEQ ID NO: 4.
14. The multiple antigen-binding antibody of item 1, wherein at least one of the two light chain variable domains is or is derived from a human Vkappa1 family light chain variable region.
15. The multiple antigen-binding antibody of item 1, wherein at least one of the two heavy chain variable domains is or is derived from a human VH3 family heavy chain variable region.
16. The multiple antigen-binding antibody of item 1, wherein the VH domains and the VL domains comprise CDRs from a lagomorph.
17. The multiple antigen-binding antibody of item 1, wherein VH-A and/or VH-B comprise Serine at AHo position 12, Threonine at AHo position 103, and Threonine at AHo position 144.
18. The multiple antigen-binding antibody of item 1, wherein the Arginine at AHo position 50 of VL-A and/or VL-B is introduced by substitution.
19. The multiple antigen-binding antibody of item 1, wherein at least one of Serine at AHo position 12, Serine or Threonine at AHo position 103, and Serine or Threonine at AHo position 144 of VH-A and/or VH-B are introduced by substitution.
20. The multiple antigen-binding antibody of item 1, wherein the antibody is bivalent.
21. The multiple antigen-binding antibody of item 1, wherein the antibody is bispecific.
22. The multiple antigen-binding antibody of item 1, wherein at least one of the heavy chain variable domains comprises at least three of the following: threonine (T) at AHo position 24, valine (V) at AHo position 25, alanine (A) or glycine (G) at AHo position 56, lysine (K) at AHo position 82, threonine (T) at AHo position 84, valine (V) at AHo position 89 and arginine (R) at AHo position 108.
23. A pharmaceutical composition comprising the multiple antigen-binding antibody of item 1.
24. Use of the multiple antigen-binding antibody of item 1 for diagnosis and/or treatment of a disease.

## Claims

1. A multiple antigen-binding antibody molecule comprising:
a) a heavy chain variable domain with a specificity for antigen A (VH-A) linked to a light chain variable domain with a specificity for antigen B (VL-B) by peptide linker 1 to form a VH-A/VL-B construct;
b) a heavy chain variable domain with a specificity for antigen B (VH-B) linked to a light chain variable domain with a specificity for antigen A (VL-A) by peptide linker 2 to form a VH-B/VL-A construct;
wherein the multiple antigen-binding antibody lacks constant domains, at least one of VL-A and VL-B comprises an Arginine at AHo position 50, and/or at least one of VH-A and VH-B comprises at least one of the following: a Serine at AHo position 12, a Serine or Threonine at AHo position 103, and a Serine or Threonine at AHo position 144;
wherein VL-A and VL-B each comprises a framework sequence that has at least 90% identity to the sequence of SEQ ID NO: 5, and
wherein VH-A and VH-B each comprises a framework sequence that has at least 90% identity to the sequence of SEQ ID NO: 6.

2. The multiple antigen-binding antibody of claim 1, wherein the VH domains and the VL domains comprise CDRs from a lagomorph.

3. The multiple antigen-binding antibody of claim 1 or 2, wherein VH-A and/or VH-B comprise Serine at AHo position 12, Threonine at AHo position 103, and Threonine at AHo position 144.

4. The multiple antigen-binding antibody of any one of claims 1-3, wherein the Arginine at AHo position 50 of VL-A and/or VL-B is introduced by substitution.

5. The multiple antigen-binding antibody of any one of claims 1-4, wherein at least one of Serine at AHo position 12, Serine or Threonine at AHo position 103, and Serine or Threonine at AHo position 144 of VH-A and/or VH-B are introduced by substitution.

6. The multiple antigen-binding antibody of any one of claims 1-5, wherein peptide linker 1 and peptide linker 2 each have 1-10 amino acids.

7. The multiple antigen-binding antibody of any one of claims 1-6, further comprising peptide linker 3 which links the VH-A/VL-B construct to the VH-B/VL-A construct, the peptide linker 3 having 10-30 amino acids.

8. The multiple antigen-binding antibody of 7, wherein peptide linker 1 and peptide linker 2 each have 3-7 amino acids, and peptide linker 3 has 15-20 amino acids.

9. The multiple antigen-binding antibody of 8, wherein peptide linker 1 and peptide linker 2 each have 5 amino acids, and peptide linker 3 has 20 amino acids.

10. The multiple antigen-binding antibody of 9, wherein peptide linker 1 comprises the sequence of GGGGS (SEQ ID NO: 1), peptide linker 2 comprises the sequence of GGGGS (SEQ ID NO: 1), and peptide linker 3 comprises the sequence of (GGGGS)₄ (SEQ ID NO: 4).

11. The multiple antigen-binding antibody of 10, having the format VH-A-SEQ ID NO: 1-VL-B-SEQ ID NO: 4-VH-B-SEQ ID NO: 1-VL-A.

12. The multiple antigen-binding antibody of any one of claims 1-11, wherein the antibody is bivalent.

13. The multiple antigen-binding antibody of any one of claims 1-12, wherein the antibody is bispecific.

14. A pharmaceutical composition comprising the multiple antigen-binding antibody of any one of claims 1-13.

15. Use of the multiple antigen-binding antibody of any one of claims 1-13 for diagnosis and/or treatment of a disease.

16. A nucleic acid molecule encoding the VH-A/VL-B construct and/or VH-B/VL-A construct of any one of claims 1-13.

17. A vector comprising the nucleic acid molecule of 16.

18. An isolated host cell comprising the vector of 17.


SEQUENCE LISTING

<110>  ESBATech - A Novartis Company LLC

<120>  STABLE MULTIPLE ANTIGEN-BINDING ANTIBODY

<130>  EP84487IFZRPpau

<140>  not yet assigned
<141>  2012-10-19

<150>  12780659.4
<151>  2012-10-19

<150>  PCT/EP2012/004404
<151>  2012-10-19

<150>  61/549,482
<151>  2011-10-20

<160>  12

<170>  PatentIn version 3.5

<210>  1
<211>  5
<212>  PRT
<213>  Artificial Sequence

<220>
<223>  synthetic peptide linker

<400>  1

Gly Gly Gly Gly Ser
1               5


<210>  2
<211>  7
<212>  PRT
<213>  Artificial Sequence

<220>
<223>  synthetic peptide linker

<400>  2

Gly Gly Gly Gly Gly Gly Ser
1               5


<210>  3
<211>  15
<212>  PRT
<213>  Artificial Sequence

<220>
<223>  synthetic peptide linker

<400>  3

Gly Ser Asp Ser Asn Ala Gly Arg Ala Ser Ala Gly Asn Thr Ser

1               5                   10                  15


<210>  4
<211>  20
<212>  PRT
<213>  Artificial Sequence

<220>
<223>  synthetic peptide linker

<400>  4

Gly Gly Gly Gly Ser Gly Gly Gly Gly Ser Gly Gly Gly Gly Ser Gly
1               5                   10                  15


Gly Gly Gly Ser
            20


<210>  5
<211>  231
<212>  PRT
<213>  Artificial Sequence

<220>
<223>  synthetic antibody framework sequence


<220>
<221>  MISC_FEATURE
<222>  (24)..(73)
<223>  CDR insertion site of 1 to 50 amino acids; X when present can be
       any amino acid

<220>
<221>  MISC_FEATURE
<222>  (89)..(138)
<223>  CDR insertion site of 1 to 50 amino acids; X when present can be
       any amino acid

<220>
<221>  MISC_FEATURE
<222>  (171)..(220)
<223>  CDR insertion site of 1 to 50 amino acids; X when present can be
       any amino acid

<400>  5

Glu Ile Val Met Thr Gln Ser Pro Ser Thr Leu Ser Ala Ser Val Gly
1               5                   10                  15


Asp Arg Val Ile Ile Thr Cys Xaa Xaa Xaa Xaa Xaa Xaa Xaa Xaa Xaa
            20                  25                  30


Xaa Xaa Xaa Xaa Xaa Xaa Xaa Xaa Xaa Xaa Xaa Xaa Xaa Xaa Xaa Xaa
        35                  40                  45


Xaa Xaa Xaa Xaa Xaa Xaa Xaa Xaa Xaa Xaa Xaa Xaa Xaa Xaa Xaa Xaa

50                  55                  60

Xaa Xaa Xaa Xaa Xaa Xaa Xaa Xaa Xaa Trp Tyr Gln Gln Lys Pro Gly
65                  70                  75                  80

Arg Ala Pro Lys Leu Leu Ile Tyr Xaa Xaa Xaa Xaa Xaa Xaa Xaa Xaa
                85                  90                  95

Xaa Xaa Xaa Xaa Xaa Xaa Xaa Xaa Xaa Xaa Xaa Xaa Xaa Xaa Xaa Xaa
            100                 105                 110

Xaa Xaa Xaa Xaa Xaa Xaa Xaa Xaa Xaa Xaa Xaa Xaa Xaa Xaa Xaa Xaa
        115                 120                 125

Xaa Xaa Xaa Xaa Xaa Xaa Xaa Xaa Xaa Xaa Gly Val Pro Ser Arg Phe
    130                 135                 140

Ser Gly Ser Gly Ser Gly Ala Glu Phe Thr Leu Thr Ile Ser Ser Leu
145                 150                 155                 160

Gln Pro Asp Asp Phe Ala Thr Tyr Tyr Cys Xaa Xaa Xaa Xaa Xaa Xaa
                165                 170                 175

Xaa Xaa Xaa Xaa Xaa Xaa Xaa Xaa Xaa Xaa Xaa Xaa Xaa Xaa Xaa Xaa
            180                 185                 190

Xaa Xaa Xaa Xaa Xaa Xaa Xaa Xaa Xaa Xaa Xaa Xaa Xaa Xaa Xaa Xaa
        195                 200                 205

Xaa Xaa Xaa Xaa Xaa Xaa Xaa Xaa Xaa Xaa Xaa Xaa Phe Gly Gln Gly
    210                 215                 220

Thr Lys Leu Thr Val Leu Gly
225                 230

<210>  6
<211>  232
<212>  PRT
<213>  Artificial Sequence

<220>
<223>  synthetic antibody framework sequence


<220>
<221>  MISC_FEATURE
<222>  (26)..(75)
<223>  CDR insertion site of 1 to 50 amino acids; X when present can be
       any amino acid

<220>

<221>  MISC_FEATURE
<222>  (90)..(139)
<223>  CDR insertion site of 1 to 50 amino acids; X when present can be
       any amino acid

<220>
<221>  MISC_FEATURE
<222>  (172)..(221)
<223>  CDR insertion site of 1 to 50 amino acids; X when present can be
       any amino acid

<400>  6

Glu Val Gln Leu Val Glu Ser Gly Gly Gly Leu Val Gln Pro Gly Gly
1               5                   10                  15

Ser Leu Arg Leu Ser Cys Thr Ala Ser Xaa Xaa Xaa Xaa Xaa Xaa Xaa
            20                  25                  30

Xaa Xaa Xaa Xaa Xaa Xaa Xaa Xaa Xaa Xaa Xaa Xaa Xaa Xaa Xaa Xaa
        35                  40                  45

Xaa Xaa Xaa Xaa Xaa Xaa Xaa Xaa Xaa Xaa Xaa Xaa Xaa Xaa Xaa Xaa
    50                  55                  60

Xaa Xaa Xaa Xaa Xaa Xaa Xaa Xaa Xaa Xaa Xaa Trp Val Arg Gln Ala
65                  70                  75                  80

Pro Gly Lys Gly Leu Glu Trp Val Gly Xaa Xaa Xaa Xaa Xaa Xaa Xaa
                85                  90                  95

Xaa Xaa Xaa Xaa Xaa Xaa Xaa Xaa Xaa Xaa Xaa Xaa Xaa Xaa Xaa Xaa
            100                 105                 110

Xaa Xaa Xaa Xaa Xaa Xaa Xaa Xaa Xaa Xaa Xaa Xaa Xaa Xaa Xaa Xaa
        115                 120                 125

Xaa Xaa Xaa Xaa Xaa Xaa Xaa Xaa Xaa Xaa Xaa Arg Phe Thr Ile Ser
    130                 135                 140

Arg Asp Thr Ser Lys Asn Thr Val Tyr Leu Gln Met Asn Ser Leu Arg
145                 150                 155                 160

Ala Glu Asp Thr Ala Val Tyr Tyr Cys Ala Arg Xaa Xaa Xaa Xaa Xaa
                165                 170                 175

Xaa Xaa Xaa Xaa Xaa Xaa Xaa Xaa Xaa Xaa Xaa Xaa Xaa Xaa Xaa Xaa
            180                 185                 190

Xaa Xaa Xaa Xaa Xaa Xaa Xaa Xaa Xaa Xaa Xaa Xaa Xaa Xaa Xaa Xaa
        195                 200                 205

Xaa Xaa Xaa Xaa Xaa Xaa Xaa Xaa Xaa Xaa Xaa Xaa Xaa Trp Gly Gln
    210                 215                 220

Gly Thr Leu Val Thr Val Ser Ser
225                 230

<210>  7
<211>  111
<212>  PRT
<213>  Artificial Sequence

<220>
<223>  synthetic light chain

<400>  7

Glu Ile Val Met Thr Gln Ser Pro Ser Thr Leu Ser Ala Ser Val Gly
1               5                   10                  15

Asp Arg Val Ile Ile Thr Cys Gln Ala Ser Glu Ile Ile His Ser Trp
            20                  25                  30

Leu Ala Trp Tyr Gln Gln Lys Pro Gly Lys Ala Pro Lys Leu Leu Ile
        35                  40                  45

Tyr Leu Ala Ser Thr Leu Ala Ser Gly Val Pro Ser Arg Phe Ser Gly
    50                  55                  60

Ser Gly Ser Gly Ala Glu Phe Thr Leu Thr Ile Ser Ser Leu Gln Pro
65                  70                  75                  80

Asp Asp Phe Ala Thr Tyr Tyr Cys Gln Asn Val Tyr Leu Ala Ser Thr
                85                  90                  95

Asn Gly Ala Asn Phe Gly Gln Gly Thr Lys Leu Thr Val Leu Gly
            100                 105                 110

<210>  8
<211>  120
<212>  PRT
<213>  Artificial Sequence

<220>
<223>  synthetic heavy chain

<400>  8

Glu Val Gln Leu Val Glu Ser Gly Gly Gly Leu Val Gln Pro Gly Gly
1               5                   10                  15

Ser Leu Arg Leu Ser Cys Thr Ala Ser Gly Phe Ser Leu Thr Asp Tyr

20                  25                  30

Tyr Tyr Met Thr Trp Val Arg Gln Ala Pro Gly Lys Gly Leu Glu Trp
        35                  40                  45

Val Gly Phe Ile Asp Pro Asp Asp Asp Pro Tyr Tyr Ala Thr Trp Ala
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Gly Gly Asp His Asn Ser Gly Trp Gly Leu Asp Ile Trp Gly Gln
            100                 105                 110

Gly Thr Leu Val Thr Val Ser Ser
        115                 120

<210>  9
<211>  120
<212>  PRT
<213>  Artificial Sequence

<220>
<223>  synthetic heavy chain

<400>  9

Glu Val Gln Leu Val Glu Ser Gly Gly Gly Ser Val Gln Pro Gly Gly
1               5                   10                  15

Ser Leu Arg Leu Ser Cys Thr Ala Ser Gly Phe Ser Leu Thr Asp Tyr
            20                  25                  30

Tyr Tyr Met Thr Trp Val Arg Gln Ala Pro Gly Lys Gly Leu Glu Trp
        35                  40                  45

Val Gly Phe Ile Asp Pro Asp Asp Asp Pro Tyr Tyr Ala Thr Trp Ala
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Gly Gly Asp His Asn Ser Gly Trp Gly Leu Asp Ile Trp Gly Gln
            100                 105                 110

Gly Thr Thr Val Thr Val Ser Ser
        115                 120

<210>  10
<211>  112
<212>  PRT
<213>  Artificial Sequence

<220>
<223>  synthetic light chain

<400>  10

Glu Ile Val Met Thr Gln Ser Pro Ser Thr Leu Ser Ala Ser Val Gly
1               5                   10                  15

Asp Arg Val Ile Ile Thr Cys Gln Ser Ser Gln Ser Val Tyr Gly Asn
            20                  25                  30

Ile Trp Met Ala Trp Tyr Gln Gln Lys Pro Gly Arg Ala Pro Lys Leu
        35                  40                  45

Leu Ile Tyr Gln Ala Ser Lys Leu Ala Ser Gly Val Pro Ser Arg Phe
    50                  55                  60

Ser Gly Ser Gly Ser Gly Ala Glu Phe Thr Leu Thr Ile Ser Ser Leu
65                  70                  75                  80

Gln Pro Asp Asp Phe Ala Thr Tyr Tyr Cys Gln Gly Asn Phe Asn Thr
                85                  90                  95

Gly Asp Arg Tyr Ala Phe Gly Gln Gly Thr Lys Leu Thr Val Leu Gly
            100                 105                 110

<210>  11
<211>  121
<212>  PRT
<213>  Artificial Sequence

<220>
<223>  synthetic heavy chain

<400>  11

Glu Val Gln Leu Val Glu Ser Gly Gly Gly Ser Val Gln Pro Gly Gly
1               5                   10                  15

Ser Leu Arg Leu Ser Cys Thr Ala Ser Gly Phe Thr Ile Ser Arg Ser
            20                  25                  30

Tyr Trp Ile Cys Trp Val Arg Gln Ala Pro Gly Lys Gly Leu Glu Trp

35                  40                  45

Val Gly Cys Ile Tyr Gly Asp Asn Asp Ile Thr Pro Leu Tyr Ala Asn
    50                  55                  60

Trp Ala Lys Gly Arg Phe Thr Ile Ser Arg Asp Thr Ser Lys Asn Thr
65                  70                  75                  80

Val Tyr Leu Gln Met Asn Ser Leu Arg Ala Glu Asp Thr Ala Thr Tyr
                85                  90                  95

Tyr Cys Ala Arg Leu Gly Tyr Ala Asp Tyr Ala Tyr Asp Leu Trp Gly
            100                 105                 110

Gln Gly Thr Thr Val Thr Val Ser Ser
        115                 120

<210>  12
<211>  495
<212>  PRT
<213>  Artificial Sequence

<220>
<223>  synthetic antibody contruct

<400>  12

Met Glu Val Gln Leu Val Glu Ser Gly Gly Gly Ser Val Gln Pro Gly
1               5                   10                  15

Gly Ser Leu Arg Leu Ser Cys Thr Ala Ser Gly Phe Ser Leu Thr Asp
            20                  25                  30

Tyr Tyr Tyr Met Thr Trp Val Arg Gln Ala Pro Gly Lys Gly Leu Glu
        35                  40                  45

Trp Val Gly Phe Ile Asp Pro Asp Asp Asp Pro Tyr Tyr Ala Thr Trp
    50                  55                  60

Ala Lys Gly Arg Phe Thr Ile Ser Arg Asp Asn Ser Lys Asn Thr Leu
65                  70                  75                  80

Tyr Leu Gln Met Asn Ser Leu Arg Ala Glu Asp Thr Ala Thr Tyr Tyr
                85                  90                  95

Cys Ala Gly Gly Asp His Asn Ser Gly Trp Gly Leu Asp Ile Trp Gly
            100                 105                 110

Gln Gly Thr Thr Val Thr Val Ser Ser Gly Gly Gly Gly Ser Glu Ile
        115                 120                 125

Val Met Thr Gln Ser Pro Ser Thr Leu Ser Ala Ser Val Gly Asp Arg
    130                 135                 140

Val Ile Ile Thr Cys Gln Ser Ser Gln Ser Val Tyr Gly Asn Ile Trp
145                 150                 155                 160

Met Ala Trp Tyr Gln Gln Lys Pro Gly Arg Ala Pro Lys Leu Leu Ile
                165                 170                 175

Tyr Gln Ala Ser Lys Leu Ala Ser Gly Val Pro Ser Arg Phe Ser Gly
            180                 185                 190

Ser Gly Ser Gly Ala Glu Phe Thr Leu Thr Ile Ser Ser Leu Gln Pro
        195                 200                 205

Asp Asp Phe Ala Thr Tyr Tyr Cys Gln Gly Asn Phe Asn Thr Gly Asp
    210                 215                 220

Arg Tyr Ala Phe Gly Gln Gly Thr Lys Leu Thr Val Leu Gly Gly Gly
225                 230                 235                 240

Gly Gly Ser Gly Gly Gly Gly Ser Gly Gly Gly Gly Ser Gly Gly Gly
                245                 250                 255

Gly Ser Glu Val Gln Leu Val Glu Ser Gly Gly Gly Ser Val Gln Pro
            260                 265                 270

Gly Gly Ser Leu Arg Leu Ser Cys Thr Ala Ser Gly Phe Thr Ile Ser
        275                 280                 285

Arg Ser Tyr Trp Ile Cys Trp Val Arg Gln Ala Pro Gly Lys Gly Leu
    290                 295                 300

Glu Trp Val Gly Cys Ile Tyr Gly Asp Asn Asp Ile Thr Pro Leu Tyr
305                 310                 315                 320

Ala Asn Trp Ala Lys Gly Arg Phe Thr Ile Ser Arg Asp Thr Ser Lys
                325                 330                 335

Asn Thr Val Tyr Leu Gln Met Asn Ser Leu Arg Ala Glu Asp Thr Ala
            340                 345                 350

Thr Tyr Tyr Cys Ala Arg Leu Gly Tyr Ala Asp Tyr Ala Tyr Asp Leu
        355                 360                 365

Trp Gly Gln Gly Thr Thr Val Thr Val Ser Ser Gly Gly Gly Gly Ser

370                 375                 380

Glu Ile Val Met Thr Gln Ser Pro Ser Thr Leu Ser Ala Ser Val Gly
385                 390                 395                 400

Asp Arg Val Ile Ile Thr Cys Gln Ala Ser Glu Ile Ile His Ser Trp
                405                 410                 415

Leu Ala Trp Tyr Gln Gln Lys Pro Gly Lys Ala Pro Lys Leu Leu Ile
            420                 425                 430

Tyr Leu Ala Ser Thr Leu Ala Ser Gly Val Pro Ser Arg Phe Ser Gly
        435                 440                 445

Ser Gly Ser Gly Ala Glu Phe Thr Leu Thr Ile Ser Ser Leu Gln Pro
    450                 455                 460

Asp Asp Phe Ala Thr Tyr Tyr Cys Gln Asn Val Tyr Leu Ala Ser Thr
465                 470                 475                 480

Asn Gly Ala Asn Phe Gly Gln Gly Thr Lys Leu Thr Val Leu Gly
                485                 490                 495